# EUROPEAN PATENT SPECIFICATION

(11) **EP 3 752 604 B1**
(45) Date of publication and mention of the grant of the patent: **04.10.2023**
(21) Application number: 19768559.7
(22) Date of filing: 18.03.2019
(51) Int. Cl.: C12N 5/09, C12Q 1/6886, G01N 33/50, G01N 1/30

(54) **METHODS FOR PERSONALIZED DETECTION OF THE RECURRENCE OF CANCER OR METASTASIS AND/OR EVALUATION OF TREATMENT RESPONSE**
VERFAHREN ZUR PERSONALISIERTEN DETEKTION DES WIEDERAUFTRETENS VON KREBS ODER METASTASEN UND/ODER AUSWERTUNG DES ANSPRECHENS AUF EINE BEHANDLUNG
PROCÉDÉS POUR LA DÉTECTION PERSONNALISÉE DE LA RÉCIDIVE D'UN CANCER OU D'UNE MÉTASTASE ET/OU L'ÉVALUATION D'UNE RÉPONSE À UN TRAITEMENT

(30) Priority: 16.03.2018 US 201862643827 P
(43) Date of publication of application: 23.12.2020
(73) Proprietor: Gopath Laboratories LLC, Buffalo Grove, Illinois 60089 (US)
(72) Inventor: LU, Jim, Buffalo Grove, Illinois 60089 (US); GUO, Zhongmin, Buffalo Grove, Illinois 60089 (US)
(74) Representative: Danner, Stefan
(86) International application number: PCT/US2019/022801
(87) International publication number: WO 2019/178606

(56) References cited:
- WO-A1-2008/151031
- WO-A1-2014/201092
- WO-A1-2016/049658
- WO-A2-2006/047787
- WO-A2-2014/089241
- WO-A2-2016/141324
- WO-A2-2016/141324
- WORST et al.: "Next-generation personalised medicine for high-risk paediatric cancer patients", Eur J Cancer, vol. 65, 29 July 2016 (2016-07-29), pages 91-101, XP029703422, DOI: doi:10.1016/j.ejca.2016.06.009
- PADOVAN-HERHAR et al.: "Enrichment of Targetable Mutations in the Relapsed Neuroblastoma Genome", PLoS Genet, vol. 12, no. 12, 20 December 2016 (2016-12-20), pages e1006501 (1)-e1006501 (13), XP055635272,
- HARTTRAMPF et al.: "Molecular Screening for Cancer Treatment Optimization (MOSCATO-01) in Pediatric Patients: A Single-Institutional Prospective Molecular Stratification Trial", Clin Cancer Res, vol. 23, no. 20, 21 July 2017 (2017-07-21) , - 15 October 2017 (2017-10-15), pages 6101-6112, XP055635279,

## Description

### CROSS REFERENCE TO RELATED APPLICATIONS

The present application claims priority to U.S. Non-Provisional Patent Application Ser. No. 16/356,693, filed March 18, 2019, which claims priority to Provisional Patent Application Ser. No. 62/643,827, filed March 16, 2018.

### FIELD OF THE INVENTION

The present invention generally relates to methods personalized detection of the recurrence of cancer or metastasis, particularly in their early stages. The present disclosure also provides methods for assessing the efficacy of an anti-cancer therapy.

### BACKGROUND

Recurrence and metastasis are major concerns for subjects being treated for cancer and for those subjects in remission. Indeed, subjects are typically at the highest risk for recurrence within the first two years following treatment. Understanding the Risk of Late Recurrence of Breast Cancer, Cancer.net, available at http://www.cancer.net/blog/2017-05/understanding-risk-late-recurrence-breast-cancer, published May 25, 2017, accessed Oct. 24, 2017. Unfortunately, a significant number of subjects will experience recurrence or metastasis.

Studies have shown that for solid cancers, metastatic spread of the cancer is responsible for more than 90% of cancer-associated deaths. Additionally, studies have shown that recurrence occurs in a number of subjects for a variety of common cancers. For example, one study has shown that approximately 30% of colorectal cancer subjects who are in remission after initial treatment will develop recurrence. Similarly, there is a 10%-30% lifetime chance of recurrence in subjects with prostate cancer after they have undergone a prostatectomy for localized cancer. For non-small cell lung cancer, recurrence occurs in approximately 50% of subjects. The rate of recurrence for subjects diagnosed with ovarian cancer is even higher at approximately 70%.

Further, minimal residual disease (MRD), which refers to small numbers of cancer cells that remain in a subject during or after treatment (even when the subject is in remission), is a significant contributor to recurrence or metastasis of hematological cancers or malignancies. Detection of MRD is critical to the management of hematological cancers or malignancies. Detecting MRD is extremely difficult and requires the use of highly sensitive tests capable of detecting minute levels of cancer (e.g., cancer cells) in tissue samples. Because of the difficulties associated with MRD detection, subjects with hematological cancer are frequently not monitored for MRD and recurrence or metastasis is not detected until the subject exhibits symptoms associated with their original hematological cancer or malignancy. Further, those subjects with MRD typically do not undergo routine screening to determine whether their MRD is stable or is progressing. As a result, these subjects must wait to see whether symptoms recur, while knowing that their MRD could lead to recurrence.

Frequently, recurrence of a cancer or metastasis is not detected until the cancer has grown to a size that is detectable by an imaging scan, such as a CT scan. Waiting until the cancer is detectable by imaging can complicate treatment, reduce a subject's treatment options, and increase the likelihood that invasive procedures will be necessary. Further, delay in diagnosing recurrence or metastasis can result in a poorer prognosis for the subject. This increases a subject's anxiety post-treatment and even post remission. Exemplary methods for the monitoring of cancer progression after onset of a treatment regimen by comparing genetic profiles taken at different time points are disclosed in WO 2016/141324, WO 2006/047787, WO 2008/151031, and WO 2014/089241. Thus, there is a need for methods that can detect recurrence or metastasis more quickly than traditional methods and that are less cumbersome and invasive for subjects.

### SUMMARY

The present i invention relates to methods for detecting the recurrence of a cancer or metastasis.

The present invention provides for a method for detecting the recurrence of a cancer in a subject as specified in claim 1. For example, the subject may have had his or her cancer diagnosed as in remission. The method comprises obtaining a tissue sample (e.g., one or more cells from a cancer) from a tumor biopsy of the subject prior to administration of an anti-cancer therapy; detecting one or more genetic mutations in the biological sample, including, for example, to generate a genetic profile for the subject; obtaining a liquid biopsy from the subject after the subject is administered an anti-cancer therapy; and detecting the one or more genetic mutations in the liquid biopsy, wherein the cancer has recurred, where the one or more mutations detected in the liquid biopsy are present in the tissue sample, including, for example, the genetic profile generated from the tissue sample. Alternatively, the cancer has not recurred, where the one or more mutations in the genetic profile are not detected in the liquid biopsy. In preferred embodiments, the liquid biopsy is obtained from the subject after the subject has been diagnosed as cancer-free (e.g., is in remission). In this manner, the method disclosed herein may be used to monitor a subject for recurrence of a cancer that had previously gone into remission. Additionally, the method may be used to monitor the effectiveness (e.g., therapeutic effectiveness) of a cancer therapy by determining whether the cancer has recurred after treatment with an anti-cancer therapy.

In some embodiments of each or any of the above or below mentioned embodiments, the cancer is determined to have recurred, where one of the mutations in the genetic profile is present in the liquid biopsy. In other embodiments, the cancer is determined to have recurred, where two, three, four, five, six, seven, eight, nine, ten, or more of the mutations in the genetic profile are present in the liquid biopsy. In other embodiments, the cancer is determined to have recurred, where all of the mutations in the genetic profile are detected in the liquid biopsy.

As disclosed herein, the tissue sample is from a cancer. In some embodiments, the tissue sample is a fresh tissue sample. In other embodiments, the tissue sample is from a tumor biopsy. In yet another embodiment, the tissue sample is a formalin-fixed paraffin embedded (FFPE) tissue sample.

As disclosed herein, the tissue sample may comprise one or more areas with different pathologic morphologies. In some embodiments, the tissue sample is obtained from one or more of the areas with the most aggressive pathologic morphology.

In some embodiments of each or any of the above or below mentioned embodiments, one or more mutations are detected in the tissue sample using next generation sequencing. In some embodiments, the one or more mutations detected in the tissue sample using next generation sequencing are used to generate a genetic profile for the subject.

In some embodiments of each or any of the above or below mentioned embodiments, the liquid biopsy is an aspirate, blood, plasma, serum, sputum, urine, or saliva. In a preferred embodiment, the liquid biopsy is a blood sample. In some embodiments, the liquid biopsy is obtained from the subject after the subject is administered an anti-cancer therapy, including, for example, a predetermined period of time after the subject is administered the anti-cancer therapy such as after the subject's cancer has been diagnosed as in remission. The liquid biopsy may be obtained from the subject every one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four or more months. In yet other embodiments, the liquid biopsy is obtained from the subject every one, two, three, four, five, six, seven, eight, nine, ten or more years. In other embodiments, the liquid biopsy is obtained from the subject at one or more predetermined periods of time, including, for example, one or more predetermined periods of time set by a physician. In some embodiments, the liquid biopsy is obtained from the subject once every three months (e.g., quarterly). In other embodiments, the liquid biopsy is obtained from the subject about once every three months for a period of two consecutive years.

In some embodiments of each or any of the above or below mentioned embodiments, the step of detecting the one or more genetic mutations in the tissue sample is performed using droplet digital PCR or next generation sequencing.

In some embodiments of each or any of the above or below mentioned embodiments, the detection of the one or more genetic mutations in the liquid biopsy is used to generate a genetic profile for the subject.

In some embodiments of each or any of the above or below mentioned embodiments, the genetic profile is generated by determining a frequency of the one or more genetic mutations in the tissue sample and selecting the one or more genetic mutations for inclusion in the genetic profile, wherein the frequency of the one or more genetic mutations is above a threshold amount. In some embodiments, the genetic profile is generated by determining a location of the one or more genetic mutations on a chromosome and selecting the one or more genetic mutations for inclusion in the genetic profile, wherein the one or more genetic mutations are located on different chromosomes.

In some embodiments of each or any of the above or below mentioned embodiments, the cancer is selected from the group consisting of gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer, colorectal cancer, renal cancer, hematological cancer, hematological disease, hematological malignancy, minimal residual disease, and bladder cancer.

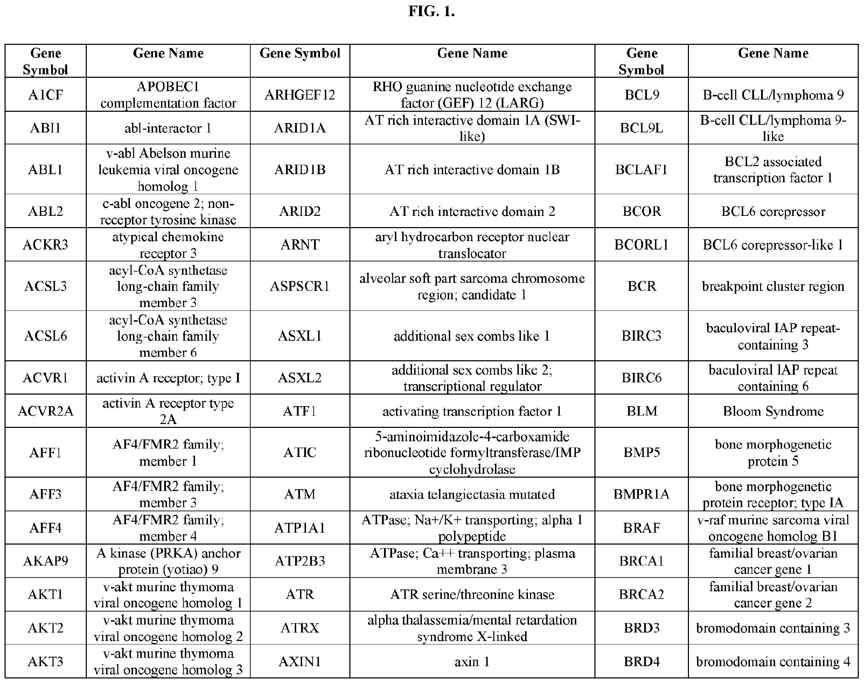

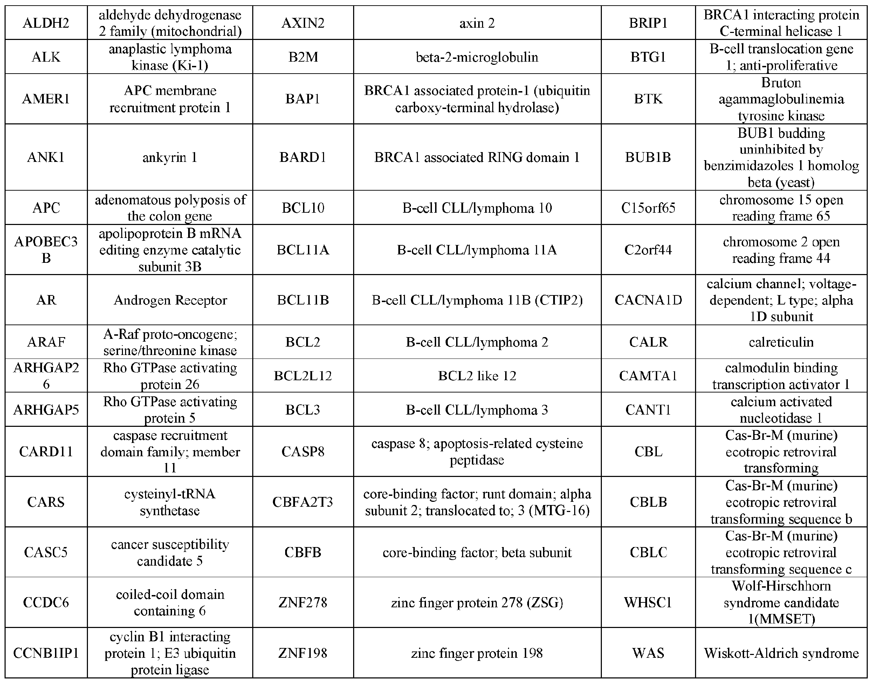

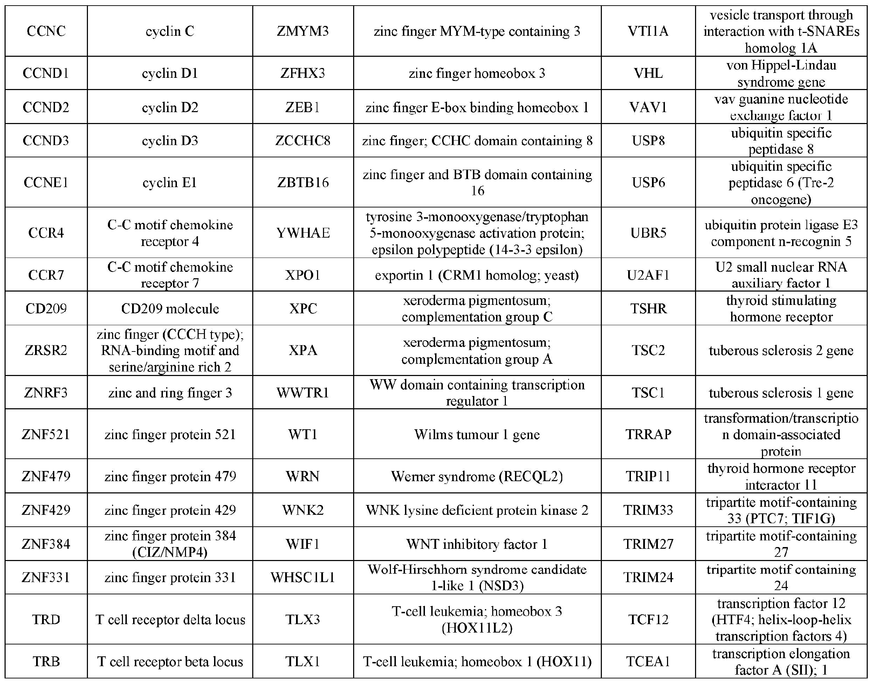

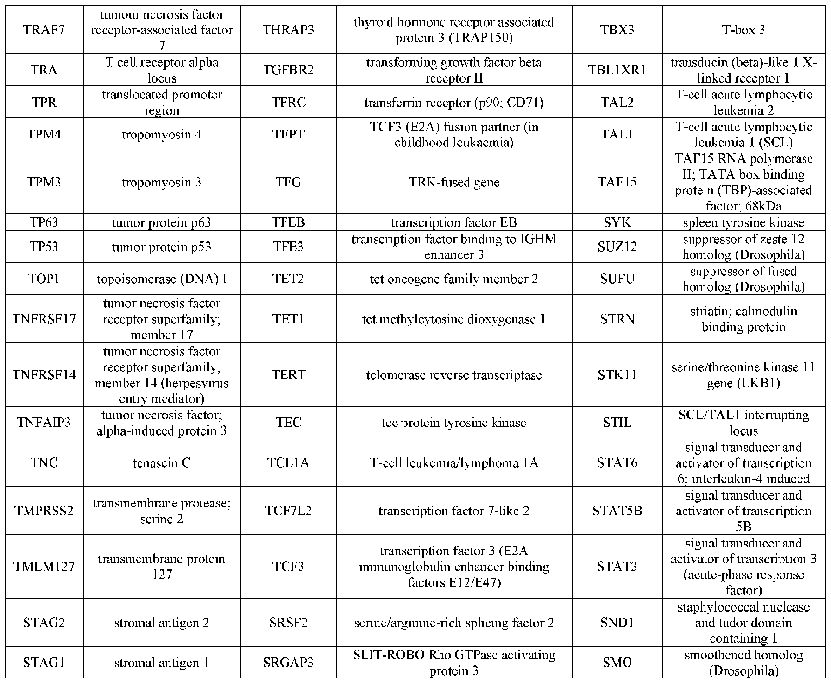

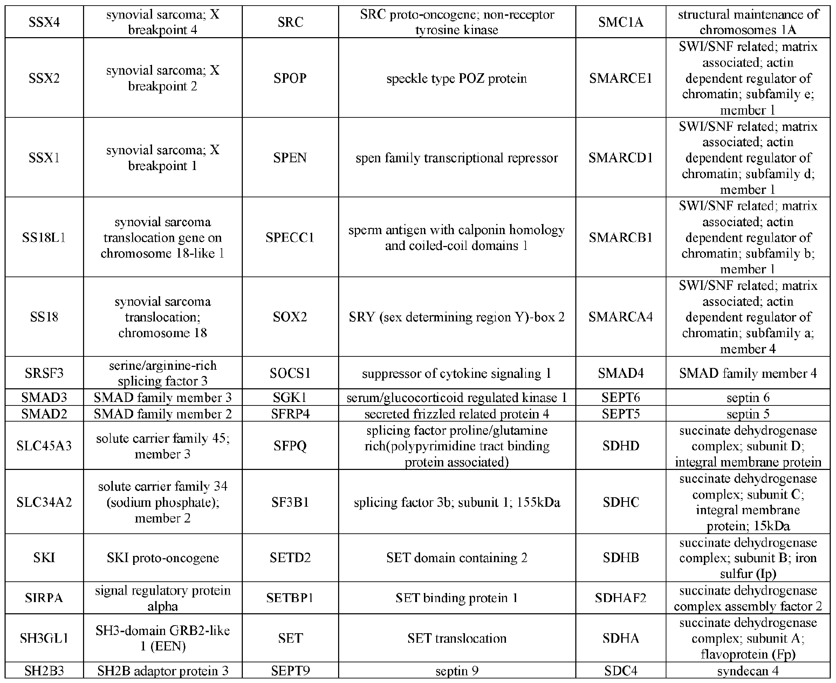

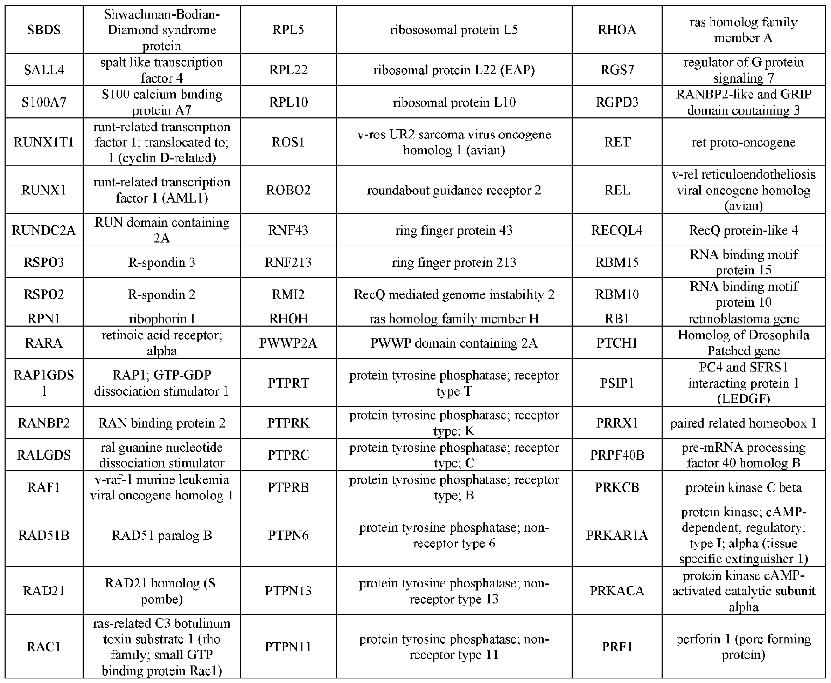

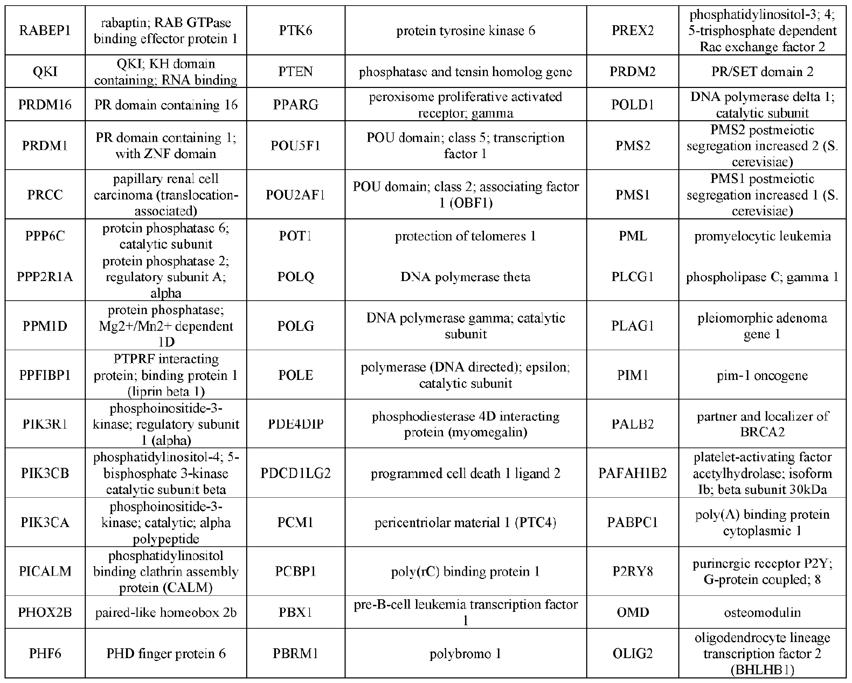

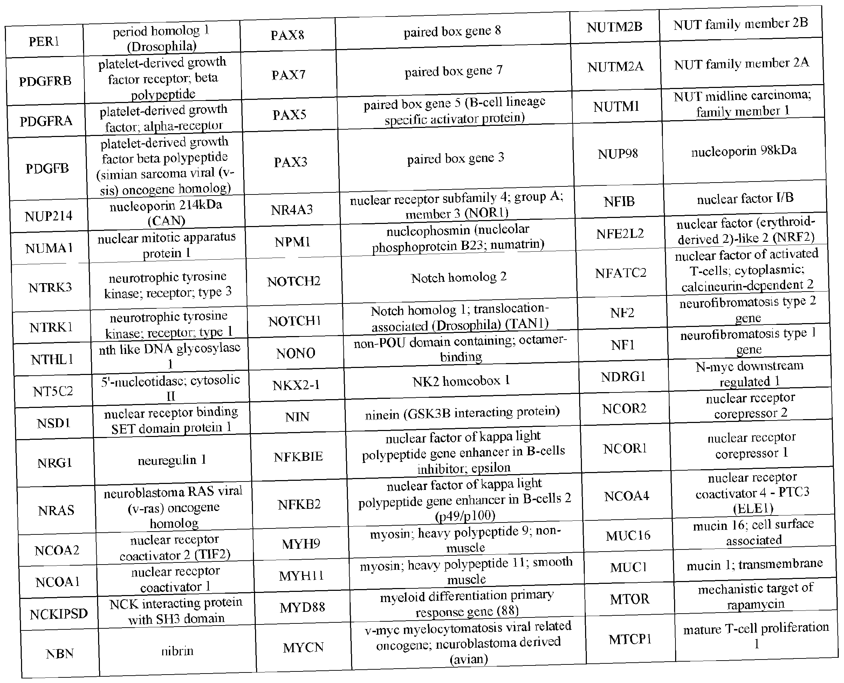

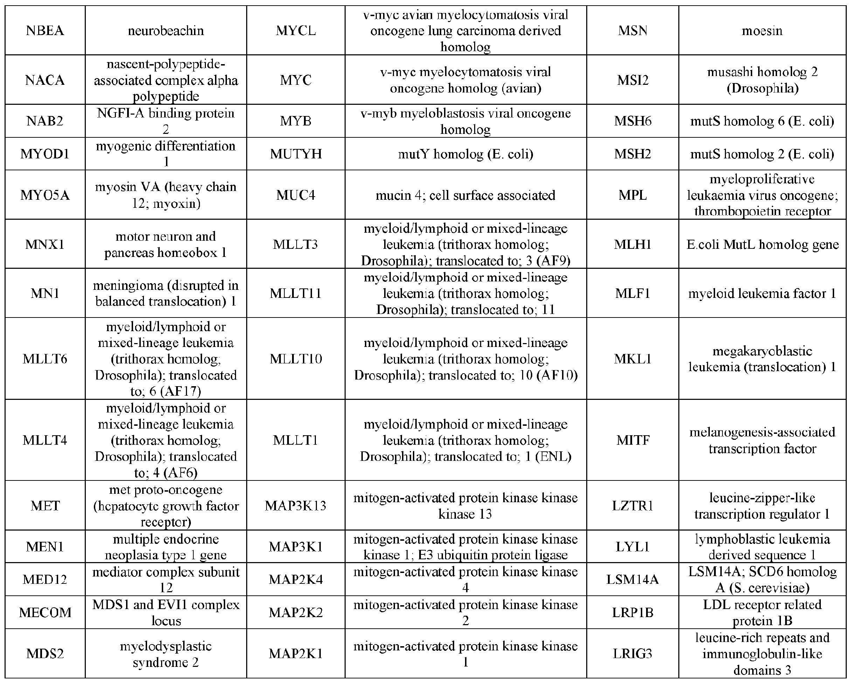

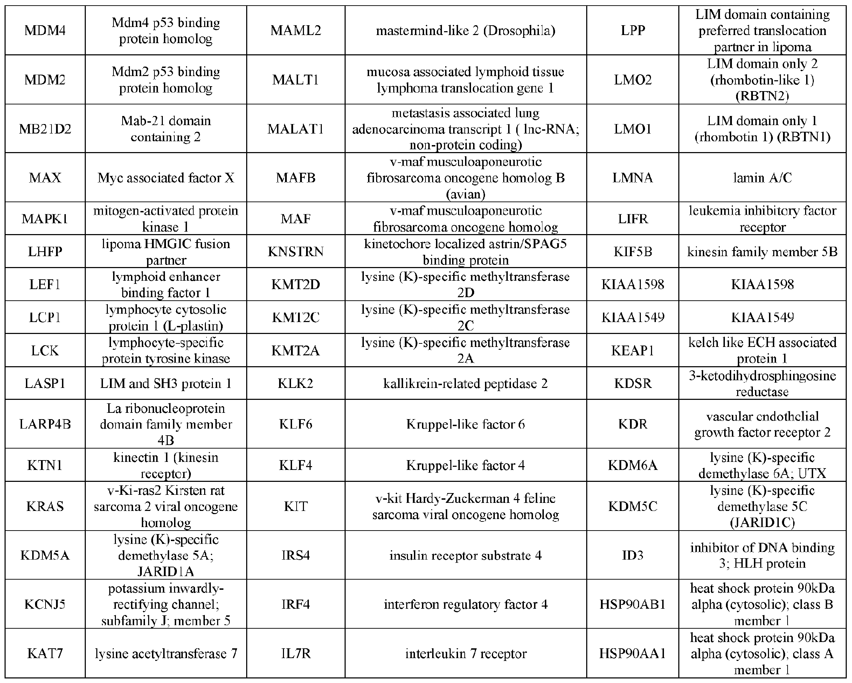

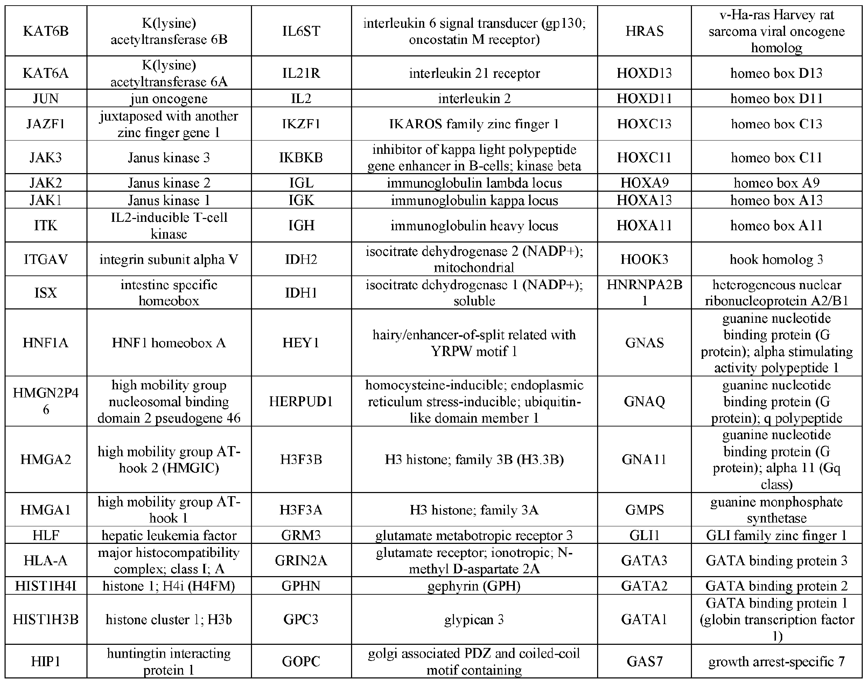

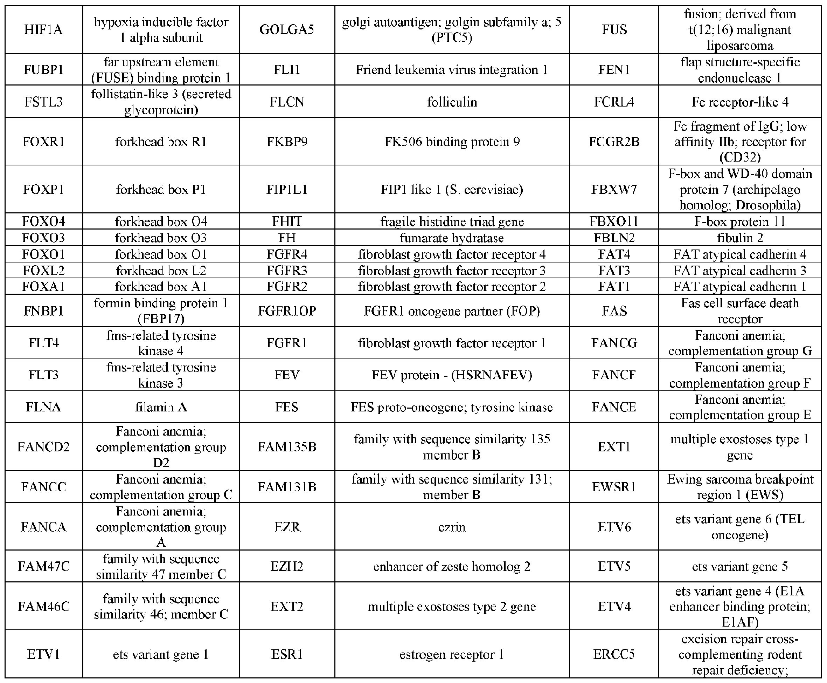

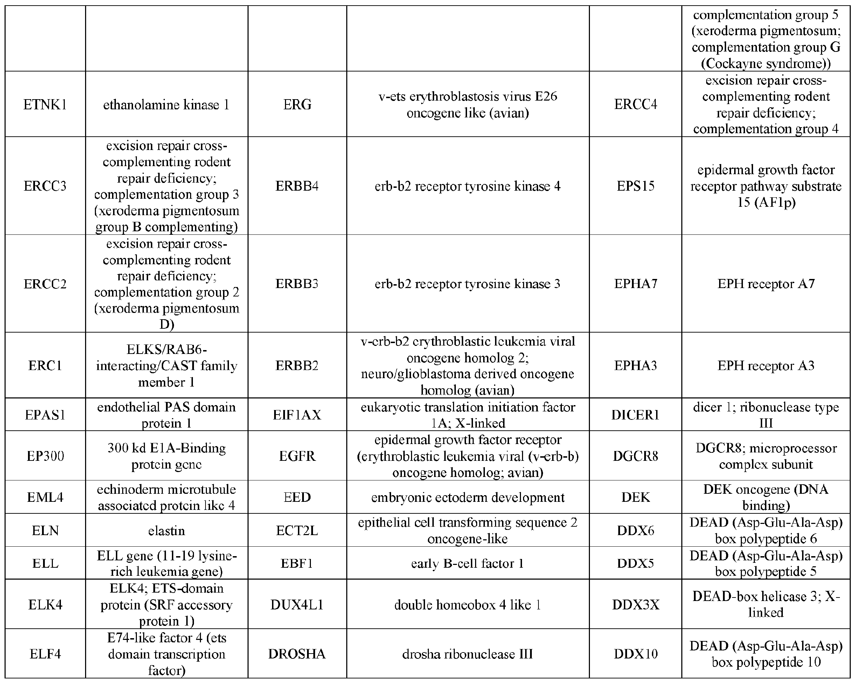

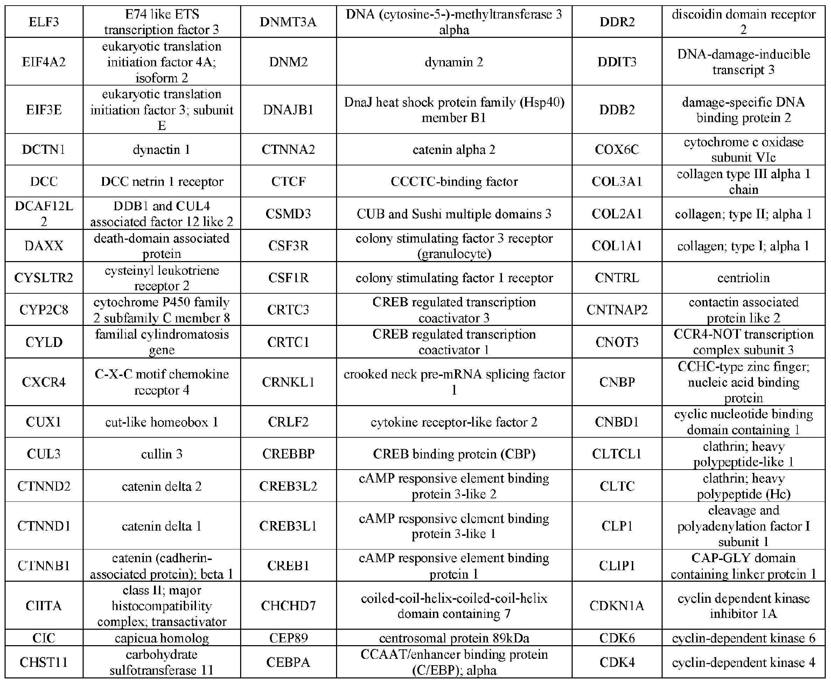

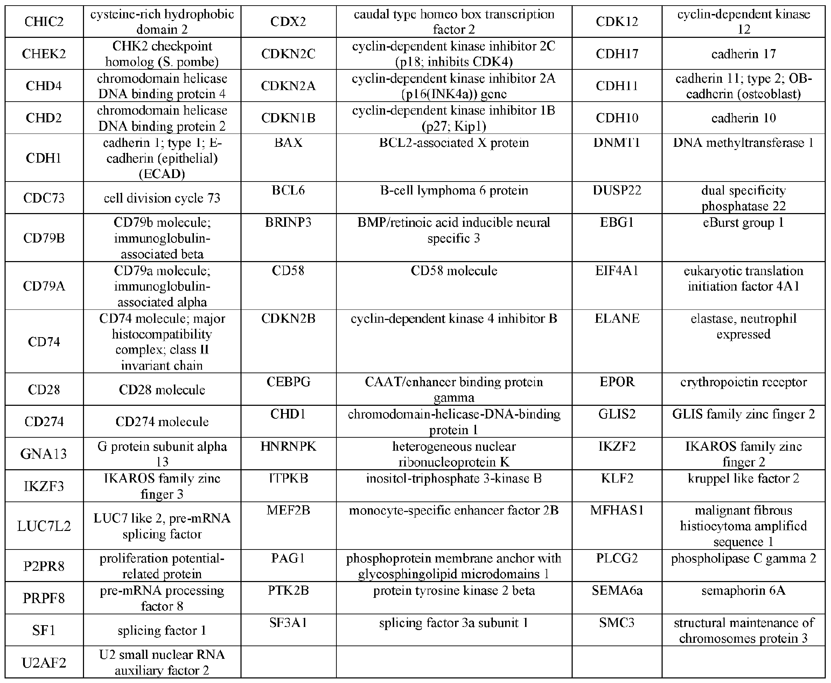

In some embodiments of each or any of the above or below mentioned embodiments, the one or more mutations are selected from those mutations set forth in FIG. 1.

The method for detecting the recurrence of a cancer in a subject, as disclosed herein, may comprise obtaining a tissue sample (e.g., one or more cells from a cancer) from a subject prior to administration of an anti-cancer therapy; detecting one or more genetic mutations in the tissue sample to generate a genetic profile for the subject; obtaining a liquid biopsy from the subject after the subject is administered an anti-cancer therapy; and detecting the one or more genetic mutations in the liquid biopsy, wherein the cancer has recurred, where the one or more mutations detected in the liquid biopsy are present in the genetic profile. Alternatively, the cancer has not recurred where the one or more mutations in the genetic profile are not detected in the liquid biopsy. In preferred embodiments, the liquid biopsy is obtained from the subject after the subject has been diagnosed as cancer-free (e.g., is in remission).

The method for detecting the recurrence of a cancer in a subject, as disclosed herein, may comprise obtaining a tissue sample (e.g., one or more cells from a cancer) from a subject prior to administration of an anti-cancer therapy; detecting one or more genetic mutations in the tissue sample to generate a genetic profile for the subject; obtaining a liquid biopsy from the subject after the subject is administered an anti-cancer therapy every three months for two consecutive years; and detecting the one or more genetic mutations in the liquid biopsy, wherein the cancer has recurred, where the one or more mutations detected in the liquid biopsy are present in the genetic profile. Alternatively, the cancer has not recurred where the one or more mutations in the genetic profile are not detected in the liquid biopsy. In preferred embodiments, the liquid biopsy is obtained from the subject after the subject has been diagnosed as cancer-free (e.g., is in remission).

The method for detecting the recurrence of a cancer in a subject, as disclosed herein, may comprise obtaining a tissue sample (e.g., one or more cells from a cancer) from a subject prior to administration of an anti-cancer therapy; detecting one or more genetic mutations in the tissue sample to generate a genetic profile for the subject; obtaining a liquid biopsy from the subject after the subject is administered an anti-cancer therapy every three months for two consecutive years; and detecting the one or more genetic mutations in the liquid biopsy, wherein the cancer has recurred, where the one or more mutations detected in the liquid biopsy are present in the genetic profile. Alternatively, the cancer has not recurred, where the one or more mutations in the genetic profile are not detected in the liquid biopsy. In preferred embodiments, the liquid biopsy is obtained from the subject after the subject has been diagnosed as cancer-free (e.g., is in remission).

method disclosed herein may also be applied for detecting a metastasis in a subject. The method comprises obtaining a tissue sample (e.g., one or more cells from a cancer) from a subject (e.g., prior to administration of an anti-cancer therapy); detecting one or more genetic mutations in the tissue sample to generate a genetic profile for the subject; obtaining a liquid biopsy from the subject (e.g., after the subject was administered an anti-cancer therapy); and detecting the one or more genetic mutations in the liquid biopsy, wherein metastasis has occurred, where the one or more mutations detected in the liquid biopsy are present in the genetic profile. Alternatively, metastasis has not occurred, where the one or more mutations in the genetic profile are not detected in the liquid biopsy. Preferably, the liquid biopsy is obtained from the subject after the subject has been diagnosed as cancer-free (e.g., is in remission). In this manner, the method disclosed herein may be used to monitor a subject for a metastasis. Additionally, the method may be used to monitor the effectiveness (e.g., therapeutic effectiveness) of a cancer therapy by determining whether a metastasis has formed after treatment with an anti-cancer therapy.

The method disclosed herein may also be applied for detecting a minimal residual cancer in a subject. The method comprises obtaining a tissue sample (e.g., one or more cells from a cancer) from a subject (e.g., prior to administration of an anti-cancer therapy); detecting one or more genetic mutations in the tissue sample to generate a genetic profile for the subject; obtaining a liquid biopsy from the subject (e.g, after the subject was administered an anti-cancer therapy); and detecting the one or more genetic mutations in the liquid biopsy, wherein a minimal residual cancer has occurred, where the one or more mutations detected in the liquid biopsy are present in the genetic profile. Alternatively, a minimal residual cancer nhas not occurred, where the one or more mutations in the genetic profile are not detected in the liquid biopsy. Preferably, the liquid biopsy is obtained from the subject after the subject has been diagnosed as cancer-free (e.g., is in remission). In this manner, the method disclosed herein may be used to monitor a subject for a minimal residual disease. Additionally, the method may be used to monitor the effectiveness (e.g., therapeutic effectiveness) of a cancer therapy by determining whether a minimal residual disease has formed after treatment with an anti-cancer therapy.

The method disclosed herein may also be applied for monitoring a subject with minimal residual cancer. The method further comprises obtaining a first liquid biopsy from the subject, generating a first genetic profile of one or more genes from the first liquid biopsy, obtaining a second liquid biopsy from the subject after a predetermined period of time (e.g., one month, two months, three months, four months, five months, six months, seven months, eight months, nine months, ten months, eleven months, twelve months, two years, three years, four years, five years, six years, seven years, eight years, nine years, ten years, eleven years, twelve years, thirteen years, fourteen years, fifteen years, sixteen years, seventeen years, eighteen years, nineteen years, twenty years), and generating a second genetic profile of the one or more genes from the second liquid biopsy, wherein the subject has relapsed (e.g., a cancer for which the subject had been treated for in the past has recurred), where one or more mutations not detected in the first genetic profile are detected in the second genetic profile. Alternatively, the subject has not relapsed, where mutations not detected in the first genetic profile are not detected in the second genetic profile. In preferred embodiments, the liquid biopsy is obtained from the subject after the subject has been diagnosed as cancer-free (e.g., is in remission). Additionally, the method may be used to monitor the effectiveness (e.g., therapeutic effectiveness) of a cancer therapy by determining whether a minimal residual disease has formed after treatment with an anti-cancer therapy.

In some embodiments of each or any of the above or below mentioned embodiments, the method further comprises the step of determining a number of one or more mutations in the one or more genes from the first liquid biopsy to generate a baseline for the detected one or more mutations. In some embodiments, the method further comprises determining a number of one or more mutations in the one or more genes from the second liquid biopsy for comparison to the baseline, wherein the subject has relapsed if the number of one of more mutations in the one or more genes from the second liquid biopsy has increased over the baseline. In other embodiments, the subject had a hematological malignancy.

Methods of treating a cancer in a subject (NO PART OF THE PRESENT INVENTION)may comprise obtaining a tissue sample of a cancer from the subject before the subject is administered a first anti-cancer therapy; detecting one or more genetic mutations in the tissue sample to generate a genetic profile for the subject before the subject is administered the first anti-cancer therapy; administering the first anti-cancer therapy to the subject; obtaining a liquid biopsy from the subject after the subject was administered the first anti-cancer therapy; detecting the one or more genetic mutations in the liquid biopsy, wherein the cancer has recurred, where the one or more mutations in the genetic profile are detected in the liquid biopsy; and administering a second anti-cancer therapy to the subject when the cancer has recurred.

Methods for administering an anti-cancer therapy to a subject with cancer (NO PART OF THE PRESENT INVENTION) may comprise obtaining a liquid biopsy from the subject after the subject was administered a first anti-cancer therapy (e.g., after the subject's cancer has gone into remission); detecting one or more genetic mutations in the liquid biopsy, wherein recurrence of the cancer has occurred, where the one or more mutations detected in the liquid biopsy are present in a genetic profile obtained from the subject prior to treatment with the anti-cancer therapy; and administering a second anti-cancer therapy to the subject once recurrence of the cancer is detected.

The first anti-cancer therapy may be the same as the second anti-cancer therapy. Alternatively, the first anti-cancer therapy may be different from the second anti-cancer therapy.

The method disclosed herein may also be applied for assessing the efficacy of an anti-cancer therapy. The method comprises obtaining a tissue sample of the cancer from the subject before the subject is administered an anti-cancer therapy, detecting one or more genetic mutations in the tissue sample to generate a genetic profile for the subject, administering the anti-cancer therapy to the subject, obtaining a liquid biopsy from the subject after the subject was administered the anti-cancer therapy, and detecting the one or more genetic mutations in the liquid biopsy, wherein the anti-cancer therapy is not effective, where the one or more mutations in the genetic profile are detected in the liquid biopsy.

### BRIEF DESCRIPTION OF THE FIGURES

Figure 1 is an exemplary listing of mutations that can be detected in a biological sample or liquid biopsy.

### DETAILED DESCRIPTION

Detection of recurrence of cancer or metastasis is critical to ensure prompt, effective cancer treatment. Early detection also reduces the invasiveness of such treatment, minimizes the number of treatments required to address recurrence or metastasis, lowers overall treatment costs, and lessens patient anxiety over the possibility of recurrence or metastasis. Current methods for detecting recurrence of cancer or metastasis rely heavily on imaging scans. As a result, the recurring or metastasizing cancer must have progressed to a point at which it can be seen on such a scan to be detected. Typically, this means that tumors must have developed and grown to a sufficient size to permit clear diagnosis. By this point, treatment options may be more complicated, longer, and more expensive. Worse yet, certain treatment options may no longer be viable options and the subject's prognosis may be worse than the initial diagnosis. Additionally, decisions regarding what cancer therapies to use may rely on the detection of genetic mutations in tumor tissues samples or specimens. However, many subjects have cancers that are inoperable or do not otherwise allow for tissue sample collection, such as the case with late stage cancer or subjects with recurrent tumors. This prevents these subjects from receiving targeted treatment for their cancer. Thus, methods are desired that can be used to detect recurrence of cancer or metastasis early on and before the development of tumors or other growths that can be seen on traditional imaging scans. Such methods are desired that are also minimally invasive and can be used to routinely monitor a subject. Further, such methods are desired that can detect genetic mutations in liquid samples for use in determining which cancer therapy or therapies should be used as well as for use in determining the efficacy of an anti-cancer therapy.

Methods are provided herein to detect the recurrence of cancer. Such methods detect genetic mutations (e.g., mutations associated with cancer) in a biological specimen including, for example, a liquid-based specimen such as blood, plasma and urine etc. Consequently, the methods may overcome the clinical limitation of tissue-based technologies. Additionally, the mutations detected with the methods provided herein may be used to inform and select an appropriate treatment regimen (e.g., targeted therapy) for a subject with cancer.

The present disclosure provides methods for detecting the recurrence of a cancer in a subject. The method comprises: obtaining a tissue sample (e.g., a FFPE tissue sample) from the subject prior to administration of an anti-cancer therapy; detecting one or more genetic mutations in the tissue sample to generate a genetic profile for the subject; obtaining a liquid biopsy (e.g., a blood sample) from the subject after the subject was administered an anti-cancer therapy (e.g., once every three months); and detecting the one or more genetic mutations in the liquid biopsy, wherein recurrence of the cancer has occurred, where the one or more mutations detected in the liquid biopsy are present in the genetic profile. Such methods may be used, *inter alia,* to monitor a patient for recurrence of a cancer or used to assess the effectiveness (e.g., therapeutic effectiveness or responsiveness) of an anti-cancer therapy.

The methods disclosed herein may also be applied for detecting a metastasis in a subject. The method comprises obtaining a tissue sample (e.g., a FFPE tissue sample) from the subject prior to administration of an anti-cancer therapy; detecting one or more genetic mutations in the tissue sample to generate a genetic profile for the subject; obtaining a liquid biopsy (e.g., a blood sample) from the subject after the subject was administered an anti-cancer therapy (e.g., once every three months); and detecting the one or more genetic mutations in the liquid biopsy, wherein metastasis has occurred, where the one or more mutations detected in the liquid biopsy are present in the genetic profile. Such methods may be used, *inter alia,* to monitor a patient for metastasis or used to assess the effectiveness (e.g., therapeutic effectiveness or responsiveness) of an anti-cancer therapy.

The methods disclosed herein may also be applied for monitoring a subject with minimal cancer. The method further comprises obtaining a first liquid biopsy from the subject, generating a first genetic profile of one or more genes from the first liquid biopsy, obtaining a second liquid biopsy from the subject after a predetermined period of time (e.g., one month, two months, three months, four months, five months, six months, seven months, eight months, nine months, ten months, eleven months, twelve months, two years, three years, four years, five years, six years, seven years, eight years, nine years, ten years, eleven years, twelve years, thirteen years, fourteen years, fifteen years, sixteen years, seventeen years, eighteen years, nineteen years, twenty years), and generating a second genetic profile of the one or more genes from the second liquid biopsy, wherein the subject has relapsed, where one or more mutations not detected in the first genetic profile are detected in the second genetic profile. Alternatively, the subject has not relapsed where mutations not detected in the first genetic profile are not detected in the second genetic profile. In preferred embodiments, the liquid biopsy is obtained from the subject after the subject has been diagnosed as cancer-free (e.g., is in remission). Additionally, the methods may be used to monitor the effectiveness (e.g., therapeutic effectiveness) of a cancer therapy by determining whether a minimal residual disease has formed after treatment with an anti-cancer therapy.

In some embodiments of each or any of the above or below mentioned embodiments, the method further comprises determining a number of one or more mutations in the one or more genes from the first liquid biopsy to generate a baseline (e.g., a reference number of mutations) for the detected one or more mutations. In some embodiments, the method further comprises determining a number of one or more mutations in the one or more genes from the second liquid biopsy for comparison to the baseline, wherein the subject has relapsed (e.g., has redeveloped their cancer) if the number of the one of more mutations in the one or more genes from the second liquid biopsy has increased over the baseline (e.g., the number of mutations in the second liquid biopsy is more than the number of mutations in the first liquid biopsy). In other embodiments, the subject had a hematological malignancy.

Methods for treating cancer in a subject (NO PART OF THE PRESENT INVENTION) may comprise obtaining a biological sample of a cancer from the subject before the subject is administered an anti-cancer therapy; detecting one or more genetic mutations in the biological sample to generate a genetic profile for the subject before the subject is administered an anti-cancer therapy; administering an anti-cancer therapy to the subject; obtaining a liquid biopsy from the subject after the subject was administered an anti-cancer therapy; detecting the one or more genetic mutations in the liquid biopsy, wherein the cancer has recurred, where the one or more mutations are detected in the liquid biopsy; and administering an anti-cancer therapy to the subject when the cancer has recurred. In some embodiments, the anti-cancer therapy administered once recurrence of the cancer is detected is different than the anti-cancer therapy administered to the subject prior to recurrence of the cancer.

Methods for administering an anti-cancer therapy to a subject with cancer(NO PART OF THE PRESENT INVENTION)may comprise obtaining a liquid biopsy from the subject after the subject is administered an anti-cancer therapy; detecting one or more genetic mutations in the liquid biopsy, wherein the cancer has recurred where the one or more mutations detected in the liquid biopsy are present in a genetic profile generated from the cancer prior to administration of the anti-cancer therapy (e.g., one or more of the genetic mutations are present in the genetic profile); and administering an anti-cancer therapy to the subject upon detection of recurrence of the cancer. In some embodiments, the anti-cancer therapy administered after detection of recurrence of the cancer is different than any anti-cancer therapy previously administered to the subject.

The methods disclosed herein may also be applied for assessing the efficacy of an anti-cancer therapy. The method comprises obtaining a tissue sample of the cancer from the subject before the subject is administered an anti-cancer therapy, detecting one or more genetic mutations in the tissue sample to generate a genetic profile for the subject, administering the first anti-cancer therapy to the subject, obtaining a liquid biopsy from the subject after the subject was administered the anti-cancer therapy, and detecting the one or more genetic mutations in the liquid biopsy, wherein the anti-cancer therapy is not effective where the one or more mutations in the genetic profile are detected in the liquid biopsy.

### Detection of the Recurrence or Metastasis of a Cancer

The inventor has found that a cancer is associated with a unique genetic signature (e.g., a unique genetic profile) that can be used to determine recurrence of that cancer and/or monitor the effectiveness of or responsiveness to an anti-cancer therapy. Surprisingly, a cancer that recurs is often associated with a genetic signature that is similar to, if not identical to, the genetic signature of the cancer that was initially treated with an anti-cancer agent. Thus, a pattern of genetic mutations identified in a biological sample such as a FFPE tissue sample (e.g., a FFPE cancer tissue sample) obtained from a subject prior to treatment with an anti-cancer agent can be used to create a genetic profile specific to the subject's cancer. The genetic profile can subsequently be used to determine whether the cancer has recurred by detection of the genetic mutations, including the same pattern of genetic mutations, in a blood sample obtained from the subject after treatment with an anti-cancer agent, including, for example, after the subject has gone into remission. In this manner, the methods disclosed herein provide a personalized approach to determining whether a cancer has recurred in a subject and optionally using that information to plot medical treatment or intervention.

The present disclosure provides methods for detecting the recurrence of a cancer in a subject. The methods may comprise obtaining a tissue sample (e.g., a FFPE tissue sample) from a subject prior to administration of an anti-cancer therapy; detecting one or more genetic mutations, including, for example, somatic mutations in the tissue sample to generate a genetic profile for the subject; obtaining a liquid biopsy such as a blood sample from the subject after the subject was administered the anti-cancer therapy including, for example, after the subject has gone in remission; and detecting the one or more genetic mutations in the liquid biopsy, wherein the cancer has recurred where the one or more mutations detected in the liquid biopsy are present in the genetic profile.

The methods disclosed herein may also be applied for detecting metastasis in a subject. The methods comprises obtaining a tissue sample from a subject prior to administration of an anti-cancer therapy; detecting one or more genetic mutations, including, for example, somatic mutations in the tissue sample to generate a genetic profile for the subject; obtaining a liquid biopsy from the subject, including, for example, after the subject was administered the anti-cancer therapy; and detecting the one or more genetic mutations in the liquid biopsy, wherein metastasis has occurred where the one or more mutations detected in the liquid biopsy are present in the genetic profile.

In some embodiments, the detection of the one or more genetic mutations in the biological sample or the liquid biopsy is used to generate a genetic profile for the subject. The genetic profile may contain information regarding the identity, copy number, and/or expression level of the detected genetic mutations.

The subject's genetic profile may be generated before the subject is administered an anti-cancer therapy to determine the genetic mutations that are attributable to the cancer before it has been exposed to anti-cancer therapies that could impact its genetic makeup and the expression of its genes. The information contained in a subject's genetic profile is unique to both the subject and the subject's cancer and allows highly-sensitive, personalized monitoring of recurrence or metastasis in a subject.

A mutation may be selected for inclusion in a genetic profile based on several criteria including, but not limited to, the frequency of the mutation in the biological sample, the chromosome represented by the mutation, and/or the pathologic morphology represented by the mutation.

A mutation may be selected for inclusion in a genetic profile based on its frequency of occurrence within a biological sample, since not every cell in the biological sample may contain the mutation. Instead, a mutation may appear in only a portion of the biological sample (e.g., some cells in the biological sample). Thus, a mutation may be selected for inclusion in a genetic profile where its frequency in the biological sample is above a threshold amount including, for example, a percentage based on those cells that contain the mutation as compared to the total (or an estimated total) number of cells in the biological sample. For example, a mutation may be selected for inclusion in a genetic profile where the frequency of the mutation is at least 5%, 10%, 15%, 20%, 25%, 30%, 35%, 40%, 45%, 50%, 55%, 60%, 65%, 70%, 75%, 80%, 85%, 90%, 95%, or 100% of the total number cells (e.g., estimated total number of cells) in the biological sample.

Additionally, a mutation may be included in a genetic profile where it represents a chromosome not represented (or underrepresented) by other mutations in the genetic profile. In this manner, mutations are preferably selected for the genetic profile to provide a representation of several chromosomes, including, for example, 2, 3, 4, 5, 6, 7, 8, 9, 10, 11, 12, 13, 14, 15, 16, 17, 18, 19, 20, 21, 22, or 23 chromosomes.

Further, the one or more mutations included in the genetic profile may be selected to represent a particular pathologic morphology. Biological samples can comprise different pathologic morphologies in different locations within the sample. Some areas may have pathologic morphologies indicative of an aggressive form of cancer or malignancy. Other areas may show less aggressive pathologic morphologies. In some embodiments, mutations are selected to represent the most aggressive pathologic morphologies in the biological sample. In other embodiments, mutations are selected that represent different (e.g., including all) pathologic morphologies.

Methods for the selection of one or more mutations for inclusion in a genetic profile may include one or more of: obtaining a biological sample, detecting one or more genetic mutations in the biological sample, determining the frequency of the one or more genetic mutations, determining the chromosomes represented by the one or more genetic mutations (e.g., identifying the chromosomes that contain the genetic mutations), and determining the pathologic morphology of the biological sample or portion of the biological sample from which the mutations originated. A mutation may be selected for inclusion in a genetic profile when: its frequency is at least 5%, 10%, 15%, 20%, 25%, 30%, 35%, 40%, 45%, 50%, 55%, 60%, 65%, 70%, 75%, 80%, 85%, 90%, of the total number cells (e.g., estimated total number of cells) in the biological sample; the chromosome represented by the mutation is not represented (or is underrepresented) by other mutations in the biological sample, and/or the mutation is present in a portion of the biological sample associated with a particular pathologic morphology (e.g., the mutation is present in cells from the most-aggressive portion of the biological sample).

A genetic profile for the subject can also be generated after the subject is administered an anti-cancer therapy. This genetic profile can be based on any mutations detected in the liquid biopsy. If a genetic profile is generated based on the results from detection of mutations in the liquid biopsy, it can be compared to the genetic profile generated from the detection of mutations in the biological sample. Changes in the detected mutations and the expression levels of those mutations can be used to determine whether cancer recurrence or metastasis has occurred.

A highly-sensitive, customized assay is created for a subject based on his or her genetic profile. The customized assay is developed to detect the specific genetic mutations that were detected in the biological sample using liquid biopsy analysis. The customized assay is developed using well known techniques, such as those discussed above with respect to genetic mutation detection and quantification.

Any known methods may be used to set an appropriate threshold level above which a mutation is determined to be present and below which a mutation is determined to not be present in a biological sample. For example, the threshold may be set at a certain number of copies of a mutation. Alternatively, the threshold may be set as a certain number of reads that detect the mutation including from next-generation sequencing (e.g., 10 or more reads). The threshold may also be set at a level of expression of the mutation.

The threshold may be set above the copy number of a mutation present in a control sample (e.g., a sample that does not contain the mutation). The threshold may also be set at an expression level present in a control sample not containing the genetic mutation. In some embodiments, the expression level of a genetic mutation must be at least 10%, 20%, 30%, 40%, 50%, 60%, 70%, 80%, 90%, 100% or greater than a control sample to determine that the genetic mutation exists in the biological sample or liquid biopsy.

In some embodiments, the one or more mutations detected in the liquid biopsy are used to generate a genetic profile. The genetic profile obtained from the liquid biopsy can be compared by any means known in the art to the genetic profile generated from the biological sample obtained from the subject prior to treatment with the anti-cancer agent.

In some embodiments, the cancer is determined to have recurred where 1, 2, 3, 4, 5, 6, 7, 8, 9, 10 or more of the mutations identified in the liquid biopsy are found in the genetic profile generated from the biological sample. In other embodiments, the cancer is determined to have recurred where all of the mutations identified in the genetic profile from the liquid biopsy are found in the genetic profile generated from the biological sample.

The biological sample (tissue sample), as used herein, can be any biological tissue obtained from a subject including a human subject. Preferably, the biological sample is from a cancer. The biological sample can be from a tumor biopsy. Additionally, the biological sample can be a freshly collected sample. The biological sample can also be a formalin fixed paraffin embedded tissue sample. The biological sample can include tissues, cells, biological fluids and isolates thereof, including, but not limited to, cells or tumor cells isolated from body samples, such as, but not limited to, smears, sputum, biopsies, secretions, saliva, cerebrospinal fluid, bile, blood, plasma serum, lymph fluid, urine and feces, or tissue which has been removed from organs, such as breast, lung, intestine, skin, cervix, prostate, and stomach.

The biological sample (tissue sample)can comprise one or more areas with different pathologic morphologies. Biological samples can comprise different pathologic morphologies in different locations within the sample. Some areas may have pathologic morphologies indicative of an aggressive form of cancer or malignancy. Other areas may show less aggressive pathologic morphologies. The biological sample may be obtained from one or more areas with the most aggressive pathologic morphology. The biological sample may also be obtained from one or more areas with the least aggressive pathologic morphology. The selection of targeted pathologic morphologies allows for targeted analysis of a biological sample. Alternatively, the biological sample may comprise several samples obtained from one or more of the different pathologic morphologies. In fact, samples for each different pathologic morphology present in a sample can be included in the biological sample, which would ensure a complete analysis of all possible sources of mutations.

The biological sample (tissue sample) can be obtained using known methods, including needle biopsy, fine need aspiration, core biopsy, image-guided biopsy, CT-guided biopsy, ultrasound-guided biopsy, MRI-guided biopsy, aspiration biopsy, surgical biopsy, excisional biopsy, incisional biopsy, punch biopsy, vacuum-assisted biopsy, bone biopsy, liver biopsy, kidney biopsy, prostate biopsy, skin biopsy, endoscopic biopsy, laparoscopic biopsy, thoracoscopic biopsy, mediastinoscopic biopsy, laparotomy, thoracotomy, or sentinel lymph node mapping and biopsy.

Once a biological sample (tissue sample) is obtained, it can be preserved for further analysis using known methods. For example, the biological sample can be embedded in formalin-fixed paraffin to create a tissue block. The biological sample can also be frozen or fixed on a slide.

A number of methods can be used to detect the presence of genetic mutations in the biological sample (tissue sample) and/or to quantitate the expression of those mutations. The genetic mutations can include known mutations of specific genes, including the genes listed in Table 1, as well as the presence or absence of an amplification of certain genes. Detection of genetic mutations can be performed at the protein level and/or the nucleic acid level. Those skilled in the art will appreciate that the methods indicated below represent some of the preferred ways in which the presence of genetic mutations, including the level of expression of those genetic mutations, can be detected and/or quantitated and in no manner limit the scope of the methods that can be employed. Those skilled in the art will also be able to determine operative and optimal assay conditions for each determination by employing routine experimentation. Such methods can include, but are not limited to, in situ hybridization (ISH), Western blots, ELISA, immunoprecipitation, immunofluorescence, flow cytometry, northern blots, Polymerase Chain Reaction (PCR), quantitative real-time PCR (qPCR), droplet digital PCR, immunocytochemistry (IHC), and next generation sequencing.

The expression level of the detected genetic mutations can be determined at the protein level or the nucleic acid level. Well known nucleic acid-based techniques for assessing expression can be used, including determining the level of the mRNA for a gene containing a mutation in the biological sample, which method can use isolated RNA. Any RNA isolation technique that does not select against the isolation of mRNA can be used. Well known techniques for isolating RNA can also be used. The isolated mRNA from a biological sample can be used in hybridization or amplification assays, including, but not limited to, Southern or Northern analyses, PCR analyses, and probe assays.

An alternative method for determining the level of a gene of interest's mRNA in a biological sample or liquid biopsy involves the process of nucleic acid amplification, e.g., by RT-PCR (the experimental embodiment set forth in Mullis, 1987, U.S. Pat. No. 4,683,202) or digital PCR, ligase chain reaction (Barany (1991) Proc. Natl. Acad. Sci. USA 88:189-193), self sustained sequence replication (Guatelli et al. (1990) Proc. Natl. Acad. Sci. USA 87:1874-1878), transcriptional amplification system (Kwoh et al. (1989) Proc. Natl. Acad. Sci. USA 86:1173-1177), Q-Beta Replicase (Lizardi et al. (1988) Bio/Technology 6:1197), rolling circle replication (Lizardi et al., U.S. Pat. No. 5,854,033) or any other nucleic acid amplification method, followed by the detection of the amplified molecules using techniques well known to those of skill in the art. The above-described detection methods are useful for the detection of nucleic acid molecules that are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

Additionally, microarrays can be used to detect the expression of a gene of interest. In particular, DNA microarrays allow for the simultaneous measurement of the expression levels of large numbers of genes. Each array consists of a reproducible pattern of capture probes attached to a solid support. Labeled RNA or DNA may be hybridized to complementary probes on the array and then detected by laser scanning. Hybridization intensities for each probe on the array are determined and converted to a quantitative value representing relative gene expression levels (*see*, *e.g*., U.S. Pat. Nos. 6,040,138, 5,800,992, 6,020,135, 6,033,860, and 6,344,316). High-density oligonucleotide arrays are particularly useful for determining the gene expression profile for a large number of RNAs in a sample.

Amplification-based assays can be used to measure the copy number of a gene of interest. In such amplification-based assays, the corresponding nucleic acid sequence of a gene of interest acts as a template in an amplification reaction (for example, PCR). In a quantitative amplification, the amount of amplification product will be proportional to the amount of template in the original sample. Comparison to appropriate controls provides a measure of the copy number of the gene of interest, corresponding to the specific probe used. The presence of a higher level of amplification product, as compared to a control, is indicative of an amplified gene of interest.

Methods of "quantitative" amplification are well known to those skilled in the art. For example, quantitative PCR involves simultaneously co-amplifying a known quantity of a control sequence using the same primers. This provides an internal standard that may be used to calibrate the PCR reaction. Detailed protocols for quantitative PCR are provided, for example, in Innis et al. (1990) PCR Protocols, A Guide to Methods and Applications, Academic Press, Inc. N.Y.

Real-time PCR (RT-PCR) is another amplification technique that can be used to determine gene copy levels or levels of mRNA expression of a gene of interest. (See, e.g., Gibson et al., Genome Research 6:995-1001, 1996; Heid et al., Genome Research 6:986-994, 1996). Real-time PCR evaluates the level of PCR product accumulation during amplification. This technique permits quantitative evaluation of mRNA levels in multiple samples. For gene copy levels, total genomic DNA is isolated from a sample. For mRNA levels, mRNA is extracted from tumor and normal tissue and cDNA is prepared using standard techniques. Real-time PCR can be performed, for example, using a Perkin Elmer/Applied Biosystems (Foster City, Calif.) 7700 Prism instrument. Matching primers and fluorescent probes can be designed for genes of interest using, for example, the primer express program provided by Perkin Elmer/Applied Biosystems (Foster City, Calif.). Optimal concentrations of primers and probes can be initially determined by those of ordinary skill in the art, and control (for example, beta-actin) primers and probes may be obtained commercially from, for example, Perkin Elmer/Applied Biosystems (Foster City, Calif.). To quantitate the amount of the specific nucleic acid of interest in a sample, a standard curve is generated using a control. Standard curves may be generated using the Ct values determined in the real-time PCR, which are related to the initial concentration of the nucleic acid of interest used in the assay. Standard dilutions ranging from 10-10⁶ copies of the gene of interest are generally sufficient. In addition, a standard curve is generated for the control sequence. This permits standardization of initial content of the nucleic acid of interest in a tissue sample to the amount of control for comparison purposes.

Droplet digital PCR is another amplification technique that can be used to determine gene copy levels or levels of mRNA expression of a gene of interest. This techinique is a digital PCR method that utilizes a water-oil emulsion droplet system. A water-oil emulsion is used to form thousands of nanoliter-sized droplets that separate the template DNA molecules. PCR amplification takes place within each individual droplet. This allows thousands of individual amplifications to be measured within a single sample and also reduces the required sample size. Droplet digital PCR uses reagents and workflows similar to those used for most TaqMan probe-based assays, which are discussed below. After PCR, each droplet is analyzed or read to determine the number of droplets containing a PCR product in the original sample. That information is analyzed using Poisson statistics to determine the target template concentration in the original sample.

Methods of real-time quantitative PCR and digital droplet PCR using TaqMan probes are well known in the art. Detailed protocols for real-time quantitative PCR are provided, for example, for RNA in Gibson et al., 1996, A novel method for real time quantitative RT-PCR. Genome Res., 10:995-1001; and for DNA in Heid et al., 1996, Real time quantitative PCR. Genome Res., 10:986-994.

A TaqMan-based assay also can be used to quantify a particular genomic region. TaqMan based assays use a fluorogenic oligonucleotide probe that contains a 5' fluorescent dye and a 3' quenching agent. The probe hybridizes to a PCR product, but cannot itself be extended due to a blocking agent at the 3' end. When the PCR product is amplified in subsequent cycles, the 5' nuclease activity of the polymerase, for example, AmpliTaq, results in the cleavage of the TaqMan probe. This cleavage separates the 5' fluorescent dye and the 3' quenching agent, thereby resulting in an increase in fluorescence as a function of amplification.

Other suitable amplification methods include, but are not limited to, ligase chain reaction (LCR) (see Wu and Wallace (1989) Genomics 4:560, Landegren et al. (1988) Science 241:1077, and Barringer et al. (1990) Gene 89:117), transcription amplification (Kwoh et al. (1989) Proc. Natl. Acad. Sci. USA 86:1173), self-sustained sequence replication (Guatelli et al. (1990) Proc. Nat. Acad. Sci. USA 87:1874), dot PCR, and linker adapter PCR, etc.

Fluorescence in situ hybridization (FISH) can also be used to determine the copy number of a gene of interest in a sample. FISH is known to those of skill in the art (see Angerer, 1987 Meth. Enzymol., 152: 649). Generally, in situ hybridization comprises the following major steps: (1) fixation of tissue or biological structure to be analyzed; (2) pre-hybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization; and (5) detection of the hybridized nucleic acid fragments.

In a typical in situ hybridization assay, cells or tissue sections are fixed to a solid support, typically a glass slide. If a nucleic acid is to be probed, the cells are typically denatured with heat or alkali. The cells are then contacted with a hybridization solution at a moderate temperature to permit annealing of labeled probes specific to the nucleic acid sequence encoding the protein. The targets (e.g., cells) are then typically washed at a predetermined stringency or at an increasing stringency until an appropriate signal to noise ratio is obtained.

The probes used in such applications are typically labeled, for example, with radioisotopes or fluorescent reporters. Preferred probes are sufficiently long, for example, from about 50, 100, or 200 nucleotides to about 1000 or more nucleotides, to enable specific hybridization with the target nucleic acid(s) under stringent conditions.

In some applications it is necessary to block the hybridization capacity of repetitive sequences. Thus, in some embodiments, tRNA, human genomic DNA, or Cot-1 DNA is used to block non-specific hybridization. Thus, in one embodiment of the present invention, the presence or absence of RAS amplification is determined by FISH.

Recurrence or metastasis of the subject's cancer can be monitored by obtaining liquid biopsy. The liquid biopsy can be obtained a predetermined period of time after the subject was administered an anti-cancer therapy. This predetermined period of time can be any desired length of time, such as one or more days or months or years. In other embodiments, the liquid biopsy is obtained from the subject every one, two, three, four, five, six, seven, eight, nine, ten, eleven, twelve, thirteen, fourteen, fifteen, sixteen, seventeen, eighteen, nineteen, twenty, twenty-one, twenty-two, twenty-three, or twenty-four months. In yet other embodiments, the liquid biopsy is obtained from the subject every one, two, three, four, five, six, seven, eight, nine, or ten years. In other embodiments, the liquid biopsy is obtained from the subject at predetermined periods of time that comprise any desired number of months or years. In some embodiments, the liquid biopsy is obtained from the subject once every three months (e.g., quarterly). In other embodiments, the liquid biopsy is obtained from the subject once every three months for a period of two years. In an embodiment, the liquid biopsy is obtained once the subject has gone into remission. In another embodiment, the liquid biopsy is obtained once the subject has completed his or her anti-cancer therapy treatment.

The liquid biopsy can be an aspirate, blood, plasma serum, sputum, urine, or saliva. Preferably, the liquid biopsy is a blood sample. The liquid biopsy can be obtained using known methods, including, but not limited to, using known phlebotomy techniques, procedures, and methods. In an embodiment, the liquid biopsy is 10 mL of a blood sample that is collected in EDTA tubes.

The liquid biopsy is analyzed to determine whether the genetic mutations contained in the subject's genetic profile are present. The customized assay developed from the subject's genetic profile is used to detect the presence of the genetic mutations. If the genetic mutations from the subject's genetic profile are detected, then cancer recurrence or metastasis has occurred. If no genetic mutations from the subject's genetic profile are detected, then cancer recurrence or metastasis has not occurred. In an embodiment, a second genetic profile is generated based on the detection of genetic mutations in the liquid biopsy. This genetic profile can optionally include information regarding the level of expression of genes containing the mutations of interest. The liquid biopsy based genetic profile can be compared to the biological sample based genetic profile to determine any differences or changes in the detected genetic mutations and optionally can determine any differences in the expression levels of the genes associated with the genetic mutations. This information can provide additional insight into whether recurrence or metastasis has occurred and, if so, can provide information regarding whether it shares the same characteristics as the initial cancer.

In an embodiment, the cancer may be selected from the group consisting of oral cancer, prostate cancer, rectal cancer, non-small cell lung cancer, lip and oral cavity cancer, liver cancer, lung cancer, anal cancer, kidney cancer, vulvar cancer, breast cancer, oropharyngeal cancer, nasal cavity and paranasal sinus cancer, nasopharyngeal cancer, urethra cancer, small intestine cancer, bile duct cancer, bladder cancer, ovarian cancer, laryngeal cancer, hypopharyngeal cancer, gallbladder cancer, colon cancer, colorectal cancer, head and neck cancer, glioma, parathyroid cancer, penile cancer, vaginal cancer, thyroid cancer, pancreatic cancer, esophageal cancer, Hodgkin's lymphoma, leukemia-related disorders, mycosis fungoides, hematological cancer, hematological disease, hematological malignancy, minimal residual disease, and myelodysplastic syndrome.

In another embodiment, the cancer is selected from the group consisting of gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer, colorectal cancer, renal cancer, and bladder cancer.

In another embodiment, the cancer may be non-small cell lung cancer, pancreatic cancer, breast cancer, ovarian cancer, colorectal cancer, or head and neck cancer. In yet another embodiment the cancer may be a carcinoma, a tumor, a neoplasm, a lymphoma, a melanoma, a glioma, a sarcoma, or a blastoma.

In one embodiment, the carcinoma may be selected from the group consisting of carcinoma, adenocarcinoma, adenoid cystic carcinoma, adenosquamous carcinoma, adrenocortical carcinoma, well differentiated carcinoma, squamous cell carcinoma, serous carcinoma, small cell carcinoma, invasive squamous cell carcinoma, large cell carcinoma, islet cell carcinoma, oat cell carcinoma, squamous carcinoma, undifferentiated carcinoma, verrucous carcinoma, renal cell carcinoma, papillary serous adenocarcinoma, merkel cell carcinoma, hepatocellular carcinoma, soft tissue carcinomas, bronchial gland carcinomas, capillary carcinoma, bartholin gland carcinoma, basal cell carcinoma, carcinosarcoma, papilloma/carcinoma, clear cell carcinoma, endometrioid adenocarcinoma, mesothelial carcinoma, metastatic carcinoma, mucoepidermoid carcinoma, cholangiocarcinoma, actinic keratoses, cystadenoma, and hepatic adenomatosis.

In another embodiment, the tumor may be selected from the group consisting of astrocytic tumors, malignant mesothelial tumors, ovarian germ cell tumors, supratentorial primitive neuroectodermal tumors, Wilms tumors, pituitary tumors, extragonadal germ cell tumors, gastrinoma, germ cell tumors, gestational trophoblastic tumors, brain tumors, pineal and supratentorial primitive neuroectodermal tumors, pituitary tumors, somatostatin-secreting tumors, endodermal sinus tumors, carcinoids, central cerebral astrocytoma, glucagonoma, hepatic adenoma, insulinoma, medulloepithelioma, plasmacytoma, vipoma, and pheochromocytoma.

In yet another embodiment, the neoplasm may be selected from the group consisting of intraepithelial neoplasia, multiple myeloma/plasma cell neoplasm, plasma cell neoplasm, interepithelial squamous cell neoplasia, endometrial hyperplasia, focal nodular hyperplasia, hemangioendothelioma, and malignant thymoma. In a further embodiment, the lymphoma may be selected from the group consisting of nervous system lymphoma, AIDS-related lymphoma, cutaneous T-cell lymphoma, non-Hodgkin's lymphoma, lymphoma, and Waldenstrom's macroglobulinemia. In another embodiment, the melanoma may be selected from the group consisting of acral lentiginous melanoma, superficial spreading melanoma, uveal melanoma, lentigo maligna melanomas, melanoma, intraocular melanoma, adenocarcinoma nodular melanoma, and hemangioma. In yet another embodiment, the sarcoma may be selected from the group consisting of adenomas, adenosarcoma, chondosarcoma, endometrial stromal sarcoma, Ewing's sarcoma, Kaposi's sarcoma, leiomyosarcoma, rhabdomyosarcoma, sarcoma, uterine sarcoma, osteosarcoma, and pseudosarcoma. In one embodiment, the glioma may be selected from the group consisting of glioma, brain stem glioma, and hypothalamic and visual pathway glioma. In another embodiment, the blastoma may be selected from the group consisting of pulmonary blastoma, pleuropulmonary blastoma, retinoblastoma, neuroblastoma, medulloblastoma, glioblastoma, and hemangiblastomas.

### Methods of Treatment (NO PART OF THE PRESENT INVENTION)

Methods of treating cancer in a subject may comprise obtaining a biological sample (e.g., a FFPE tissue sample) of a cancer from the subject before the subject is administered a first anti-cancer therapy; detecting one or more genetic mutations in the biological sample to generate a genetic profile for the subject before the subject is administered the first anti-cancer therapy; administering the first anti-cancer therapy to the subject; obtaining a liquid biopsy from the subject after the subject was administered the first anti-cancer therapy; detecting the one or more genetic mutations in the liquid biopsy, wherein the cancer has recurred where the mutations detected in the liquid biopsy are present in the genetic profile; and administering a second anti-cancer therapy to the subject after recurrence of the cancer.

As used herein, "treating" or "treatment" of a disease, disorder, or condition includes, at least partially, (1) preventing the disease, disorder, or condition, i.e., causing the clinical symptoms of the disease, disorder, or condition not to develop in a mammal that is exposed to or predisposed to the disease, disorder, or condition but does not yet experience or display symptoms of the disease, disorder, or condition; (2) inhibiting the disease, disorder, or condition, i.e., arresting or reducing the development of the disease, disorder, or condition or its clinical symptoms; or (3) relieving the disease, disorder, or condition, i.e., causing regression of the disease, disorder, or condition or its clinical symptoms. The treating or treatment of a disease or disorder may include treating or the treatment of cancer.

The term "treating cancer" refers to administration to a mammal afflicted with a cancerous condition and refers to an effect that alleviates the cancerous condition by killing the cancerous cells, but also to an effect that results in the inhibition of growth and/or metastasis of the cancer.

The second anti-cancer therapy may be different than the first anti-cancer therapy.

The anti-cancer therapy can include any well known therapies to treat cancer, including surgical removal of the cancer, administration of chemotherapy, administration of radiation, administration of antibody therapies, and administration of anti-cancer drugs.

The term "chemotherapy" refers to the treatment of cancer or a disease or disorder caused by a virus, bacterium, other microorganism, or an inappropriate immune response using specific chemical agents, drugs, or radioactive agents that are selectively toxic and destructive to malignant cells and tissues, viruses, bacteria, or other microorganisms. Chemotherapeutic agents or drugs such as an anti-folate (e.g., Methotrexate) or any other agent or drug useful in treating cancer, an inflammatory disease, or an autoimmune disease are preferred. Suitable chemotherapeutic agents and drugs include actinomycin D, adriamycin, altretamine, azathioprine, bleomycin, busulphan, capecitabine, carboplatin, carmustine, chlorambucil, cisplatin, cladribine, crisantaspase, cyclophosphamide, cytarabine, dacarbazine, daunorubicin, doxorubicin, epirubicin, etoposide, fludarabine, fluorouracil, gemcitabine, hydroxyurea, idarubicin, ifosfamide, irinotecan, liposomal doxorubicin, lomustine, melphalan, mercaptopurine, Methotrexate, mitomycin, mitozantrone, oxaliplatin, paclitaxel, pentostatin, procarbazine, raltitrexed, steroids, streptozocin, taxol, taxotere, temozolomide, thioguanine, thiotepa, tomudex, topotecan, treosulfan, uft (uracil-tegufur), vinblastine, vincristine, vindesine, and vinorelbine.

Methods for administering an anti-cancer therapy to a subject with cancer may comprise obtaining a liquid biopsy from the subject after a predetermined period of time after the subject was administered a first anti-cancer therapy (e.g., after the subject's cancer has gone into remission); detecting one or more genetic mutations in the liquid biopsy, wherein recurrence of the cancer has occurred where the one or more mutations detected in the liquid biopsy are present in a genetic profile created from information obtained from the subject prior to treatment with the first anti-cancer therapy; and administering a second anti-cancer therapy to the subject after recurrence of the cancer is detected.

This disclosure is further illustrated by the following examples which are provided to facilitate the practice of the disclosed methods.

### EXAMPLES

### Example 1: Detection of Recurrence of Ovarian Cancer

A FFPE tissue sample was obtained from an ovarian tumor in a female subject age 42. The FFPE tissue sample was analyzed as described herein for genetic mutations by defined next generation sequencing panels and used to create a genetic profile for the subject. Mutations in the TP53, BRCA1, and BRCA2 genes were detected. The detection of these mutations and/or the expression levels of these genes were used to generate a genetic profile for the subject before she was administered an anti-cancer therapy. The subject was then administered an anti-cancer therapy comprising a combination of a platinum compound, such as cisplatin or carboplatin, and a taxane, such as paclitaxel (Taxol^{®}) or docetaxel (Taxotere^{®}) and targeted therapy. Subsequently, after treatment, a blood sample was obtained from the subject. The blood sample was taken three months after the subject received the anti-cancer therapy. The TP53, BRCA1, and BRCA2 genetic mutations were detected in the liquid biopsy, establishing that the subject's cancer had recurred. As a result, the subject was again administered an anti-cancer therapy. In this case, the anti-cancer therapy was different than that previously administered.

## Claims

1. A method for detecting the recurrence of a cancer in a subject, the method comprising prior to administration of an anti-cancer therapy:
(a) obtaining a tissue sample from a tumor biopsy of the subject; and
(b) detecting one or more genetic mutations in the tissue sample to generate a genetic profile for the subject;
and the method comprising after administration of an anti-cancer therapy:
(c) determining that the cancer is in remission;
(d) obtaining a liquid biopsy from the subject at a predetermined period of time after administering the anti-cancer therapy; and
(e) detecting the one or more genetic mutations in the liquid biopsy,
wherein the cancer has recurred where the one or more mutations detected in the liquid biopsy are present in the genetic profile.

2. The method of claim 1, wherein the tissue sample is a formalin fixed paraffin embedded tissue sample.

3. The method of claim 1, wherein the tissue sample comprises one or more areas with different pathologic morphologies, and particularly wherein the tissue sample is obtained from one or more of the areas with the most aggressive pathologic morphology.

4. The method of claim 1, wherein the liquid biopsy is an aspirate, blood, plasma serum, sputum, urine, or saliva, and particularly wherein the liquid biopsy is blood.

5. The method of claim 1, wherein the step of detecting the one or more genetic mutations in the tissue sample is performed using droplet digital PCR or next generation sequencing.

6. The method of claim 1, wherein the liquid biopsy is obtained from the subject every one, two, or three months; or wherein the liquid biopsy is obtained from the subject every three months for two consecutive years.

7. The method of claim 1, wherein the one or more mutations are selected from the group consisting of: A1CF, AB11 ABL1.ABL2,ACKR3, ACSL3, ACSL6,ACVR1, ACVR2A, AFF1, AFF3, AFF4, AKAP9, AKT1,AKT2, AKT3, ARHGEF12, ARID1A, ARID1B, ARID2, ARNT, APSCR1, ASKL1, ASXL2, ATF1, ATF2, ATIC, ATM, ATP1A1, ATP2B3, ATR, ATRX, AXIN1, BCL9, BCL9L, BCLAF1, BCOR, BCORL1, BCR, BIRC3, BIRC6, BLM, BMP5, BMPR1A, BRAF, BRCA1, BRCA2, BRD3, BRD4, ALDH2, ALK, AMER1, ANK1, APC, APOBEC3B, AR, ARAF, ARHGAP26, ARHGAP5, CARD11, CARS, CASC5, CCDC6, CCNB11 P1, AXIN2, B2M, BAP1, BARD1, BCL10, BCL11A, BCL11B, BCL2, BCL2L12, BCL3, CASP8, CBFA2T3, CBFB, ZNF278, ZNF198, BRIP1,BTG1, BTK, BUB1B, C15ORF65, C2ORF44, CACNA1D, CALR, CAMTA1, CANT1, CBL, CBLB, CBLC, WHSC1, WAS, CCNC, CCND1, CCND2, CCND3, CCNE1, CCR4, CCR7, CD209, ZRSR2, ZNRF3, ZNF521, ZNF479, ZNF384, ZNF311, TRD, TRB, ZMYM3, ZFHX3, ZEB1, ZCCHC8, ZBTB16, YWHAE, XPO1, XPC, XPA, WWTR1, WT1, WRN, WNK2, WIF1, WHSCIL1, TLX3, TLX1, VT11A, VHL, VAV1, USP8, USP6, UBR5, U2AF1, TSHR, TSC2, TSC1, TRRAP, TRIP11, TRIM33, TRIM 27, TRIM 24, TCF12, TCEA1, TRAF7, TRA, TPR, TPM4, TPM3, TP63, TP53, TOP1, TNFRSF17, TNFRSF14, TNFAIP3, TNC, TMPRSS2, TMEM127, STAG2, STAG1, THRAP3, TGFBR2, TFRC, TFPT, TFG, TFEB, TFE3, TET2, TET1, TERT, TEC, TCL1A, TCF7L2, TCF3, SRSF2, SRGAP3, TBX3, TBL1XR1, TAL2, TAL1, TAF15, SYK, SUZ12, SUFU, STRN, STK11, STIL, STAT6, STAT5B, STST3, SBD1, SMO,SSX4, SSX2, SSX1, SS18L1, SS18, SRSF3, SMAD3, SMAD2, SLC45A3, SLC34A2, SK1, SIRPA, SH3GL1, SH2B3, SRC, SPOP, SPEN, SPECC1, SOX2, SOCS1, SGK1, SFRP4, SFPQ, SF3B1, SETD2, SETBP1, SET, SEPT9, SMC1A, SMARCEI, SMARCD1, SMARCB1, SMARCA4, SMAD4, SEPT6, SEPT5, SDHD, SDHC, SDHB, SDHAF2, SDHA, SDC4, SBDS, SALL4, S100A7, RUNX1T1, RUNX1, RUNDC2A, RSPO3, RSPO2, RPM1, RARA,RAP1GDS1, RANBP2, RALGDS, RAF1, RAD51B, RAD21, RAC1, RPL5, RPL22, RPL10, ROS1, ROBO2, RNF43, RNF213, RM12, RHOH, PWWP2A, PTPRT, PTPRK, PTPRC, PTPRB, PTPN6, PTPN13, PTPN11, RHOA, RGS7, RGPD3, RET, REL, RECQL4, RBM15, RBM10, RB1, PTCH1, PSIP1, PRRX1, PRPF40B, PRKCB, PRKAR1A, PRKACA, PRF1, RABEP1, QKI, PRDM16, PRDM1, PRCC, PPP6C, PPP2R1A, PPM1D, PPFIBP1, PIK3R1, PIK3CB, PIK3CA, PICALM, PHOX2B, PHF6, PTK6, PTEN, PPARG, POU5F1, POU2AF1, POT1, POLO, POLG, POLE, PDE4DIP, PDCD1LG2, PCM1, PCBP1, PBX1, PBRM1, PREX2, PRDM2, POLD1, PMS2, PMS1, PML, PLCG1, PLAG1, PIM1, PALB2, PAFAH1B2, PABPC1, P2RY8, OMD, OLIG2, PER1, PDGFRB, PDGFRA, PDGFB, NUP214, NUMA1, NTRK3, NTRK1, NTHL1, NT5C2, NSD1, NRG1, NRAS, NCOA2, NCOA1, NCKIPSD, NBN, PAX8, PAX7, PAX5, PAX3, NR4A3, NPM1, NOTCH2, NOTCH1, NONO, NKX2-1, NIN, NFKB1E, NFKB2, MYH9, MYH11, MYD88, MACN, NUTM2B, NUTM2A, NUTM1, NUP98, NFIB, NFE2L2, NFATC2, NF2, NF1, NDRG1, NCOR2, NCOR1, NCOA4, MUC1, MTOR, MTCP1, NBEA, NACA, NAB2, MYOD1, MYO5A, MNX1, MN1, MLLT6, MLLT4, MET, MEN1, MED12, MECOM, MDS2, MYCL, MYC, MYB, MUTYH, MUC4, MLLT3, MLLT11, MLLT10, MLLT1, MAP3K13, MAP3K1, MAP2K4, MAP2K2, MAP2K1, MSN, MS12, MSH6, MSH2, MPL, MLH1, MLF1, MKL1, MITF, LZTRI, LYL1, LSM14A, LRP1B, LRIG3, MDM4, MDM2, MB21D2, MAX, MAPK1, LHFP, LEF1, LCP1, LCK, LASP1, LARP4B, KTN1, KRAS, KDM5A, KCN15, KAT7, MAML2, MALT1, MALAT1, MAFB, MAF, KNSTRN, KMT2D, KMT2C, KMT2A, KLK2, KLF6, KLF4, KIT, IRS4, IRF4, IL7R, LPP, LMO2, LMO1, LMNA, LIFR, KIF5B, KIAA1598, KIAA1549, KEAP1, KDSR, KDR, KDM6A, KDMSC, ID3, HSP90AB1, HSP90AA1, KAT6A, JUN, JAZF1, JAK3, JAK2, JAK1, ITK, ITGAV, ISX, HNFIA, HMGN2P46, HMGA2, HMGA1, HLF, HLA-A, HIST1H4I, HIST1 H3B, HIP1, IL6ST,IL21R, IL2, IKZF1, IKBKB, IGL,IGK, IGH, IDH2, IDH1, HEY1, HERPUD1, H3F3B, H3F3A, GRM3, GRIN2A, GPHN, GPC3, GOPC, HRAS, HOXD13, HOXD11, HOXC13, HOXC11, HOXA9, HOXA13, HOXA11, HOOK3, HNRNPA2B1, GNAS, GNAQ, GNA11, GMPS, GL11, GATA3, GATA2, GATA1, GAS7, HIF1A, FUBP1, FSTL3, FOXR1, FOXP1, FOXO4, FOXO3, FOXO1, FOXL2, FOXA1, FNBP1, FLT4, FLT3, FLNA, FANCD2, FANCC, FANCA, FAM47C, FAM46C, ETV1, GOLGA5, FL11, FLCN, FKBP9, FIP1L1, FHIT, FH, FGFR4, FGFR3, FGFR2, FGFR10P, FGFR1, FEV, FES, FAM135B, FAM 131B, EZR, EZH2, EXT2, ESR1, FUS, FEN1, FCRL4, FCGR2B, FBXW7, FBXO11, FBLN2, FAT4, FAT3, FAT1, FAS, FANCG, FANCF, FANCE, EXT1, EWSR1, ETV6, ETV5, ETV4, ERCC5, ETNK1, ERCC3, ERCC2, ERC1, EPAS1, EP300, EML4, ELN, ELL, ELK4, ELF4, ERG, ERBB4, ERBB3, ERBB2, EIF1AX, EGFR, EED, ECT2L, EBF1, DUX4L1, DROSHA, ERCC4, EPS15, EPHA7, EPHA3, DICER1, DGCR8, DEK, DDX6, DDX5, DDX3X, DDX10, ELF3, EIF4A2, EIF3E, DCTN1, DCC, DCAF12L2, DAXX, CYSLTR2, CYP2C8, CYLD, CXCR4, CUX1, CUL3, CTNND2, CTNND1, CTNNB1, CIITA, CIC, CHST11, DNMT3A, DNM2, DAAJB1, CTNNA2, CTCF, CSMD3, CSF3R, CSF1R, CRTC3, CRTC1, CRNKL1, CRLF2, CREBPB, CREB3L2, CREB3L1, CREB1, CHCHD7, CEP89, CEBPA, DDR2, DDIT3, DDB2, COX6C, COL3A1, COL2A1, COL1A1, CNTRL, CNTNAP2, CNOT3, CNBP, CNBD1, CLTCL1, CLTC, CLP1, CLIP1, CDKN1A, CDK6, CDK4, CHIC2, CHEK2, CHD4, CHD2, CHD11, CDC73, CD79B, CD79A, CD74, CD28, CD274, GNA13, IKZF3, LUC7L2, P2PR8, PRPF8, SF1, U2AF2, CDX2, CDKN2C, CDKN2A, CDKN1B, BAX, BCL6, BRINP3, CD58, CDKN2B, CEBPG, CHD1, HNRNPK, ITPKB, MEF2B, PAG1, PTK2B, SF3A1, CDK12, CDH17, CDH11, CDH10, DNMT1, DUSP22, EBG1, EIF4A1, ELANE, EPOR, GLIS2, IKZF2, KLF2, MFHAS1, PLCG2, SEMA6A, AND SMC3.

8. The method of claim 1, wherein the cancer is selected from the group consisting of: gastrointestinal cancer, prostate cancer, ovarian cancer, breast cancer, head and neck cancer, lung cancer, non-small cell lung cancer, cancer of the nervous system, kidney cancer, retina cancer, skin cancer, liver cancer, pancreatic cancer, genital-urinary cancer, colorectal cancer, renal cancer, hematological cancer, hematological disease, hematological malignancy, minimal residual disease, and bladder cancer.

9. The method of claim 1, wherein the genetic profile is generated by:
(a) determining a frequency of the one or more genetic mutations in the tissue sample; and
(b) selecting the one or more genetic mutations for inclusion in the genetic profile, wherein the frequency of the one or more genetic mutations is above a threshold amount;
or wherein the genetic profile is generated by:
(a) determining a location of the one or more genetic mutations on a chromosome; and
(b) selecting the one or more genetic mutations for inclusion in the genetic profile, wherein the one or more genetic mutations are located on different chromosomes.

## Patentansprüche

1. Verfahren zum Detektieren des Wiederkehrens von Krebs in einer Testperson, wobei das Verfahren vor der Verabreichung einer Antikrebstherapie umfasst:
(a) Erhalten einer Gewebeprobe aus seiner Tumorbiopsie der Testperson; und
(b) Detektieren von einer oder mehr genetischen Mutationen in der Gewebeprobe, um ein genetisches Profil für die Testperson zu erzeugen;
und wobei das Verfahren nach der Verabreichung einer Antikrebstherapie umfasst:
(c) Bestimmen, dass sich der Krebs in Remission befindet;
(d) Erhalten einer Flüssigbiopsie der Testperson nach einer vorher festgelegten Zeitspanne nach der Verabreichung der Antikrebstherapie; und
(e) Detektieren der einen oder mehr genetischen Mutationen in der Flüssigbiopsie;
wobei der Krebs wiedergekehrt ist, wenn die eine oder mehr Mutationen, die in der Flüssigbiopsie detektiert wurden, in dem genetischen Profil enthalten sind.

2. Verfahren gemäß Anspruch 1, wobei die Gewebeprobe eine mit Formalin fixierte, in Paraffin eingebettete Gewebeprobe ist.

3. Verfahren gemäß Anspruch 1, wobei die Gewebeprobe einen oder mehr Bereiche mit unterschiedlicher pathologischer Morphologie aufweist, und insbesondere wobei die Gewebeprobe von einem oder mehr Bereichen mit der aggressivsten pathologischen Morphologie erhalten wird.

4. Verfahren gemäß Anspruch 1, wobei die Flüssigbiopsie ein Punktat, Blut, Plasma, Serum, Sputum, Urin oder Speichel ist, und insbesondere wobei die Flüssigbiopsie Blut ist.

5. Verfahren gemäß Anspruch 1, wobei der Schritt des Detektierens der einen oder mehr Mutationen in der Gewebeprobe unter Verwendung von Droplet Digital PCR oder Next Generation Sequencing durchgeführt wird.

6. Verfahren gemäß Anspruch 1, wobei die Flüssigbiopsie von der Testperson jeden, alle zwei oder alle drei Monate erhalten wird; oder wobei die Flüssigbiopsie von der Testperson alle drei Monate über zwei aufeinanderfolgende Jahre erhalten wird.

7. Verfahren gemäß Anspruch 1, wobei die eine oder mehr Mutationen aus der Gruppe ausgewählt werden, die besteht aus: A1CF, AB11 ABL1.ABL2,ACKR3, ACSL3, ACSL6,ACVR1, ACVR2A, AFF1, AFF3, AFF4, AKAP9, AKT1,AKT2, AKT3, ARHGEF12,ARID1A, ARID1B, ARID2, ARNT, APSCR1, ASKL1, ASXL2, ATF1, ATF2, ATIC, ATM, ATP1A1, ATP2B3, ATR, ATRX, AXIN1, BCL9, BCL9L, BCLAF1, BCOR, BCORL1, BCR, BIRC3, BIRC6, BLM, BMP5, BMPR1A, BRAF, BRCA1, BRCA2, BRD3, BRD4, ALDH2, ALK, AMER1, ANK1, APC, APOBEC3B, AR, ARAF, ARHGAP26, ARHGAP5, CARD11, CARS, CASC5, CCDC6, CCNB11P1, AXIN2, B2M, BAP1, BARD1, BCL10, BCL11A, BCL11B, BCL2, BCL2L12, BCL3, CASP8, CBFA2T3, CBFB, ZNF278, ZNF198, BRIP1,BTG1, BTK, BUB1B, C15ORF65, C2ORF44, CACNA1D, CALR, CAMTA1, CANT1, CBL, CBLB, CBLC, WHSC1, WAS, CCNC, CCND1, CCND2, CCND3, CCNE1, CCR4, CCR7, CD209, ZRSR2, ZNRF3, ZNF521, ZNF479, ZNF384, ZNF311, TRD, TRB, ZMYM3, ZFHX3, ZEB1, ZCCHC8, ZBTB16, YWHAE, XPO1, XPC, XPA, WWTR1, WT1, WRN, WNK2, WIF1, WHSCIL1, TLX3, TLX1, VT11A, VHL, VAV1, USP8, USP6, UBR5, U2AF1, TSHR, TSC2, TSC1, TRRAP, TRIP11, TRIM33, TRIM 27, TRIM 24, TCF12, TCEA1, TRAF7, TRA, TPR, TPM4, TPM3, TP63, TP53, TOP1, TNFRSF17, TNFRSF14, TNFAIP3, TNC, TMPRSS2, TMEM127, STAG2, STAG1, THRAP3, TGFBR2, TFRC, TFPT, TFG, TFEB, TFE3, TET2, TET1, TERT, TEC, TCL1A, TCF7L2, TCF3, SRSF2, SRGAP3, TBX3, TBL1XR1, TAL2, TAL1, TAF15, SYK, SUZ12, SUFU, STRN, STK11, STIL, STAT6, STAT5B, STST3, SBD1, SMO,SSX4, SSX2, SSX1, SS18L1, SS18, SRSF3, SMAD3, SMAD2, SLC45A3, SLC34A2, SK1, SIRPA, SH3GL1, SH2B3, SRC, SPOP, SPEN, SPECC1, SOX2, SOCS1, SGK1, SFRP4, SFPQ, SF3B1, SETD2, SETBP1, SET, SEPT9, SMC1A, SMARCEI, SMARCD1, SMARCB1, SMARCA4, SMAD4, SEPT6, SEPT5, SDHD, SDHC, SDHB, SDHAF2, SDHA, SDC4, SBDS, SALL4, S100A7, RUNX1T1, RUNX1, RUNDC2A, RSPO3, RSPO2, RPM1, RARA,RAP1GDS1, RANBP2, RALGDS, RAF1, RAD51B, RAD21, RAC1, RPL5, RPL22, RPL10, ROS1, ROBO2, RNF43, RNF213, RM12, RHOH, PWWP2A, PTPRT, PTPRK, PTPRC, PTPRB, PTPN6, PTPN13, PTPN11, RHOA, RGS7, RGPD3, RET, REL, RECQL4, RBM15, RBM10, RB1, PTCH1, PSIP1, PRRX1, PRPF40B, PRKCB, PRKAR1A, PRKACA, PRF1, RABEP1, QKI, PRDM16, PRDM1, PRCC, PPP6C, PPP2R1A, PPM1D, PPFIBP1, PIK3R1, PIK3CB, PIK3CA, PICALM, PHOX2B, PHF6, PTK6, PTEN, PPARG, POU5F1, POU2AF1, POT1, POLQ, POLG, POLE, PDE4DIP, PDCD1LG2, PCM1, PCBP1, PBX1, PBRM1, PREX2, PRDM2, POLD1, PMS2, PMS1, PML, PLCG1, PLAG1, PIM1, PALB2, PAFAH1B2, PABPC1, P2RY8, OMD, OLIG2, PER1, PDGFRB, PDGFRA, PDGFB, NUP214, NUMA1, NTRK3, NTRK1, NTHL1, NT5C2, NSD1, NRG1, NRAS, NCOA2, NCOA1, NCKIPSD, NBN, PAX8, PAX7, PAX5, PAX3, NR4A3, NPM1, NOTCH2, NOTCH1, NONO, NKX2-1, NIN, NFKB1E, NFKB2, MYH9, MYH11, MYD88, MACN, NUTM2B, NUTM2A, NUTM1, NUP98, NFIB, NFE2L2, NFATC2, NF2, NF1, NDRG1, NCOR2, NCOR1, NCOA4, MUC1, MTOR, MTCP1, NBEA, NACA, NAB2, MYOD1, MYO5A, MNX1, MN1, MLLT6, MLLT4, MET, MEN1, MED12, MECOM, MDS2, MYCL, MYC, MYB, MUTYH, MUC4, MLLT3, MLLT11, MLLT10, MLLT1, MAP3K13, MAP3K1, MAP2K4, MAP2K2, MAP2K1, MSN, MS12, MSH6, MSH2, MPL, MLH1, MLF1, MKL1, MITF, LZTRI, LYL1, LSM14A, LRP1B, LRIG3, MDM4, MDM2, MB21D2, MAX, MAPK1, LHFP, LEF1, LCP1, LCK, LASP1, LARP4B, KTN1, KRAS, KDM5A, KCN15, KAT7, MAML2, MALT1, MALAT1, MAFB, MAF, KNSTRN, KMT2D, KMT2C, KMT2A, KLK2, KLF6, KLF4, KIT, IRS4, IRF4, IL7R, LPP, LMO2, LMO1, LMNA, LIFR, KIF5B, KIAA1598, KIAA1549, KEAP1, KDSR, KDR, KDM6A, KDMSC, ID3, HSP90AB1, HSP90AA1, KAT6A, JUN, JAZF1, JAK3, JAK2, JAK1, ITK, ITGAV, ISX, HNFIA, HMGN2P46, HMGA2, HMGA1, HLF, HLA-A, HIST1H4I, HIST1H3B, HIP1, IL6ST,IL21R, IL2, IKZF1, IKBKB, IGL,IGK, IGH, IDH2, IDH1, HEY1, HERPUD1, H3F3B, H3F3A, GRM3, GRIN2A, GPHN, GPC3, GOPC, HRAS, HOXD13, HOXD11, HOXC13, HOXC11, HOXA9, HOXA13, HOXA11, HOOK3, HNRNPA2B1, GNAS, GNAQ, GNA11, GMPS, GL11, GATA3, GATA2, GATA1, GAS7, HIF1A, FUBP1, FSTL3, FOXR1, FOXP1, FOXO4, FOXO3, FOXO1, FOXL2, FOXA1, FNBP1, FLT4, FLT3, FLNA, FANCD2, FANCC, FANCA, FAM47C, FAM46C, ETV1, GOLGA5, FL11, FLCN, FKBP9, FIP1L1, FHIT, FH, FGFR4, FGFR3, FGFR2, FGFR10P, FGFR1, FEV, FES, FAM135B, FAM 131B, EZR, EZH2, EXT2, ESR1, FUS, FEN1, FCRL4, FCGR2B, FBXW7, FBXO11, FBLN2, FAT4, FAT3, FAT1, FAS, FANCG, FANCF, FANCE, EXT1, EWSR1, ETV6, ETV5, ETV4, ERCC5, ETNK1, ERCC3, ERCC2, ERC1, EPAS1, EP300, EML4, ELN, ELL, ELK4, ELF4, ERG, ERBB4, ERBB3, ERBB2, EIF1AX, EGFR, EED, ECT2L, EBF1, DUX4L1, DROSHA, ERCC4, EPS15, EPHA7, EPHA3, DICER1, DGCR8, DEK, DDX6, DDX5, DDX3X, DDX10, ELF3, EIF4A2, EIF3E, DCTN1, DCC, DCAF12L2, DAXX, CYSLTR2, CYP2C8, CYLD, CXCR4, CUX1, CUL3, CTNND2, CTNND1, CTNNB1, CIITA, CIC, CHST11, DNMT3A, DNM2, DAAJB1, CTNNA2, CTCF, CSMD3, CSF3R, CSF1R, CRTC3, CRTC1, CRNKL1, CRLF2, CREBPB, CREB3L2, CREB3L1, CREB1, CHCHD7, CEP89, CEBPA, DDR2, DDIT3, DDB2, COX6C, COL3A1, COL2A1, COL1A1, CNTRL, CNTNAP2, CNOT3, CNBP, CNBD1, CLTCL1, CLTC, CLP1, CLIP1, CDKN1A, CDK6, CDK4, CHIC2, CHEK2, CHD4, CHD2, CHD11, CDC73, CD79B, CD79A, CD74, CD28, CD274, GNA13, IKZF3, LUC7L2, P2PR8, PRPF8, SF1, U2AF2, CDX2, CDKN2C, CDKN2A, CDKN1B, BAX, BCL6, BRINP3, CD58, CDKN2B, CEBPG, CHD1, HNRNPK, ITPKB, MEF2B, PAG1, PTK2B, SF3A1, CDK12, CDH17, CDH11, CDH10, DNMT1, DUSP22, EBG1, EIF4A1, ELANE, EPOR, GLIS2, IKZF2, KLF2, MFHAS1, PLCG2, SEMA6A und SMC3.

8. Verfahren gemäß Anspruch 1, wobei der Krebs aus der Gruppe ausgewählt wird, die besteht aus: Magen-Darmkrebs, Prostatakrebs, Eierstockkrebs, Brustkrebs, Kopf-Hals-Karzinom, Lungenkrebs, nicht-kleinzelliger Lungenkrebs, Krebs des Nervensystems, Nierenkrebs, Retinakrebs, Hautkrebs, Leberkrebs, Pankreaskrebs, Urogenitalkrebs, kolorektales Karzinom, Nierenkrebs, hämatologisches Karzinom, hämatologische Erkrankung, hämatologischer bösartiger Tumor, minimale Resterkrankung und Blasenkrebs.

9. Verfahren gemäß Anspruch 1, wobei das genetische Profil erzeugt wird durch:
(a) Bestimmen der Frequenz der einen oder mehr genetischen Mutationen in der Gewebeprobe; und
(b) Selektieren der einen oder mehr genetischen Mutationen zur Aufnahme in das genetische Profil, wobei die Frequenz der einen oder mehr genetischen Mutationen oberhalb eines Schwellenwerts liegt;
oder wobei das genetische Profil erzeugt wird durch:
(a) Bestimmen der Lokalisation der einen oder mehr genetischen Mutationen auf einem Chromosom; und
(b) Selektieren der einen oder mehr genetischen Mutationen zur Aufnahme in das genetische Profil, wobei die eine oder mehr genetischen Mutationen auf verschiedenen Chromosomen lokalisiert sind.

## Revendications

1. Procédé de détection de la récidive d'un cancer chez un sujet, la méthode comprenant avant l'administration d'une thérapie anticancéreuse:
(a) obtenir un échantillon de tissu à partir d'une biopsie tumorale du sujet; et
(b) détecter une ou plusieurs mutations génétiques dans l'échantillon de tissu pour générer un profil génétique pour le sujet;
et la méthode comprenant après administration d'une thérapie anticancéreuse :
(c) déterminer que le cancer est en rémission;
(d) obtenir une biopsie liquide du sujet à une période de temps prédéterminée après l'administration de la thérapie anticancéreuse; et
(e) détecter la ou les mutations génétiques dans la biopsie liquide,
dans lequel le cancer a récidivé lorsque la ou les mutations détectées dans la biopsie liquide sont présentes dans le profil génétique.

2. Procédé selon la revendication 1, dans lequel l'échantillon de tissu est un échantillon de tissu fixé au formol et inclus en paraffine.

3. Procédé selon la revendication 1, dans lequel l'échantillon de tissu comprend une ou plusieurs zones présentant des morphologies pathologiques différentes, et en particulier dans lequel l'échantillon de tissu est obtenu à partir d'une ou plusieurs des zones présentant la morphologie pathologique la plus agressive.

4. Procédé selon la revendication 1, dans lequel la biopsie liquide est un liquide aspiré, du sang, du sérum, du plasma, des crachats, de l'urine ou de la salive, et en particulier dans lequel la biopsie liquide est du sang.

5. Procédé selon la revendication 1, dans lequel l'étape de détection de la ou des mutations génétiques dans l'échantillon de tissu est réalisée en utilisant une droplet digital PCR ou séquençage de nouvelle génération.

6. Procédé selon la revendication 1, dans lequel la biopsie liquide est obtenue du sujet tous les un, deux ou trois mois; ou dans lequel la biopsie liquide est obtenue du sujet tous les trois mois pendant deux années consécutives.

7. Procédé selon la revendication 1, dans lequel la ou les mutations sont sélectionnées dans le groupe constitué deA1CF, AB11 ABL 1.ABL2,ACKR3, ACSL3, ACSL6,ACVR1, ACVR2A, AFF1, AFF3, AFF4, AKAP9, AKT1,AKT2, AKT3, ARHGEF12, ARID1A, ARID1B, ARID2, ARNT, APSCR1, ASKL1, ASXL2, ATF1, ATF2, ATIC, ATM, ATP1A1, ATP2B3, ATR, ATRX, AXIN1, BCL9, BCL9L, BCLAF1, BCOR, BCORL1, BCR, BIRC3, BIRC6, BLM, BMP5, BMPR1A, BRAF, BRCA1, BRCA2, BRD3, BRD4, ALDH2, ALK, AMER1, ANK1, APC, APOBEC3B, AR, ARAF, ARHGAP26, ARHGAP5, CARD11, CARS, CASC5, CCDC6, CCNB11P1, AXIN2, B2M, BAP1, BARD1, BCL10, BCL11A, BCL11B, BCL2, BCL2L12, BCL3, CASP8, CBFA2T3, CBFB, ZNF278, ZNF198, BRIP1,BTG1, BTK, BUB1B, C15ORF65, C2ORF44, CACNA1D, CALR, CAMTA1, CANT1, CBL, CBLB, CBLC, WHSC1, WAS, CCNC, CCND1, CCND2, CCND3, CCNE1, CCR4, CCR7, CD209, ZRSR2, ZNRF3, ZNF521, ZNF479, ZNF384, ZNF311, TRD, TRB, ZMYM3, ZFHX3, ZEB1, ZCCHC8, ZBTB16, YWHAE, XPO1, XPC, XPA, WWTR1, WT1, WRN, WNK2, WIF1, WHSCIL1, TLX3, TLX1, VT11A, VHL, VAV1, USP8, USP6, UBR5, U2AF1, TSHR, TSC2, TSC1, TRRAP, TRIP11, TRIM33, TRIM 27, TRIM 24, TCF12, TCEA1, TRAF7, TRA, TPR, TPM4, TPM3, TP63, TP53, TOP1, TNFRSF17, TNFRSF14, TNFAIP3, TNC, TMPRSS2, TMEM127, STAG2, STAG1, THRAP3, TGFBR2, TFRC, TFPT, TFG, TFEB, TFE3, TET2, TET1, TERT, TEC, TCL1A, TCF7L2, TCF3, SRSF2, SRGAP3, TBX3, TBL1XR1, TAL2, TAL1, TAF15, SYK, SUZ12, SUFU, STRN, STK11, STIL, STAT6, STAT5B, STST3, SBD1, SMO,SSX4, SSX2, SSX1, SS18L1, SS18, SRSF3, SMAD3, SMAD2, SLC45A3, SLC34A2, SK1, SIRPA, SH3GL1, SH2B3, SRC, SPOP, SPEN, SPECC1, SOX2, SOCS1, SGK1, SFRP4, SFPQ, SF3B1, SETD2, SETBP1, SET, SEPT9, SMC1A, SMARCEI, SMARCD1, SMARCB1, SMARCA4, SMAD4, SEPT6, SEPT5, SDHD, SDHC, SDHB, SDHAF2, SDHA, SDC4, SBDS, SALL4, S100A7, RUNX1T1, RUNX1, RUNDC2A, RSPO3, RSPO2, RPM1, RARA,RAP1GDS1, RANBP2, RALGDS, RAF1, RAD51B, RAD21, RAC1, RPL5, RPL22, RPL10, ROS1, ROBO2, RNF43, RNF213, RM12, RHOH, PWWP2A, PTPRT, PTPRK, PTPRC, PTPRB, PTPN6, PTPN13, PTPN11, RHOA, RGS7, RGPD3, RET, REL, RECQL4, RBM15, RBM10, RB1, PTCH1, PSIP1, PRRX1, PRPF40B, PRKCB, PRKAR1A, PRKACA, PRF1, RABEP1, QKI, PRDM16, PRDM1, PRCC, PPP6C, PPP2R1A, PPM1D, PPFIBP1, PIK3R1, PIK3CB, PIK3CA, PICALM, PHOX2B, PHF6, PTK6, PTEN, PPARG, POU5F1, POU2AF1, POT1, POLQ, POLG, POLE, PDE4DIP, PDCD1LG2, PCM1, PCBP1, PBX1, PBRM1, PREX2, PRDM2, POLD1, PMS2, PMS1, PML, PLCG1, PLAG1, PIM1, PALB2, PAFAH1B2, PABPC1, P2RY8, OMD, OLIG2, PER1, PDGFRB, PDGFRA, PDGFB, NUP214, NUMA1, NTRK3, NTRK1, NTHL1, NT5C2, NSD1, NRG1, NRAS, NCOA2, NCOA1, NCKIPSD, NBN, PAX8, PAX7, PAX5, PAX3, NR4A3, NPM1, NOTCH2, NOTCH1, NONO, NKX2-1, NIN, NFKB1E, NFKB2, MYH9, MYH11, MYD88, MACN, NUTM2B, NUTM2A, NUTM1, NUP98, NFIB, NFE2L2, NFATC2, NF2, NF1, NDRG1, NCOR2, NCOR1, NCOA4, MUC1, MTOR, MTCP1, NBEA, NACA, NAB2, MYOD1, MYO5A, MNX1, MN1, MLLT6, MLLT4, MET, MEN1, MED12, MECOM, MDS2, MYCL, MYC, MYB, MUTYH, MUC4, MLLT3, MLLT11, MLLT10, MLLT1, MAP3K13, MAP3K1, MAP2K4, MAP2K2, MAP2K1, MSN, MS12, MSH6, MSH2, MPL, MLH1, MLF1, MKL1, MITF, LZTRI, LYL1, LSM14A, LRP1B, LRIG3, MDM4, MDM2, MB21D2, MAX, MAPK1, LHFP, LEF1, LCP1, LCK, LASP1, LARP4B, KTN1, KRAS, KDM5A, KCN15, KAT7, MAML2, MALT1, MALAT1, MAFB, MAF, KNSTRN, KMT2D, KMT2C, KMT2A, KLK2, KLF6, KLF4, KIT, IRS4, IRF4, IL7R, LPP, LMO2, LMO1, LMNA, LIFR, KIF5B, KIAA1598, KIAA1549, KEAP1, KDSR, KDR, KDM6A, KDMSC, ID3, HSP90AB1, HSP90AA1, KAT6A, JUN, JAZF1, JAK3, JAK2, JAK1, ITK, ITGAV, ISX, HNFIA, HMGN2P46, HMGA2, HMGA1, HLF, HLA-A, HIST1H4I, HIST1H3B, HIP1, IL6ST,IL21R, IL2, IKZF1, IKBKB, IGL,IGK, IGH, IDH2, IDH1, HEY1, HERPUD1, H3F3B, H3F3A, GRM3, GRIN2A, GPHN, GPC3, GOPC, HRAS, HOXD13, HOXD11, HOXC13, HOXC11, HOXA9, HOXA13, HOXA11, HOOK3, HNRNPA2B1, GNAS, GNAQ, GNA11, GMPS, GL11, GATA3, GATA2, GATA1, GAS7, HIF1A, FUBP1, FSTL3, FOXR1, FOXP1, FOXO4, FOXO3, FOXO1, FOXL2, FOXA1, FNBP1, FLT4, FLT3, FLNA, FANCD2, FANCC, FANCA, FAM47C, FAM46C, ETV1, GOLGA5, FL11, FLCN, FKBP9, FIP1L1, FHIT, FH, FGFR4, FGFR3, FGFR2, FGFR10P, FGFR1, FEV, FES, FAM135B, FAM 131B, EZR, EZH2, EXT2, ESR1, FUS, FEN1, FCRL4, FCGR2B, FBXW7, FBXO11, FBLN2, FAT4, FAT3, FAT1, FAS, FANCG, FANCF, FANCE, EXT1, EWSR1, ETV6, ETV5, ETV4, ERCC5, ETNK1, ERCC3, ERCC2, ERC1, EPAS1, EP300, EML4, ELN, ELL, ELK4, ELF4, ERG, ERBB4, ERBB3, ERBB2, EIF1AX, EGFR, EED, ECT2L, EBF1, DUX4L1, DROSHA, ERCC4, EPS15, EPHA7, EPHA3, DICER1, DGCR8, DEK, DDX6, DDX5, DDX3X, DDX10, ELF3, EIF4A2, EIF3E, DCTN1, DCC, DCAF12L2, DAXX, CYSLTR2, CYP2C8, CYLD, CXCR4, CUX1, CUL3, CTNND2, CTNND1, CTNNB1, CIITA, CIC, CHST11, DNMT3A, DNM2, DAAJB1, CTNNA2, CTCF, CSMD3, CSF3R, CSF1R, CRTC3, CRTC1, CRNKL1, CRLF2, CREBPB, CREB3L2, CREB3L1, CREB1, CHCHD7, CEP89, CEBPA, DDR2, DDIT3, DDB2, COX6C, COL3A1, COL2A1, COL1A1, CNTRL, CNTNAP2, CNOT3, CNBP, CNBD1, CLTCL1, CLTC, CLP1, CLIP1, CDKN1A, CDK6, CDK4, CHIC2, CHEK2, CHD4, CHD2, CHD11, CDC73, CD79B, CD79A, CD74, CD28, CD274, GNA13, IKZF3, LUC7L2, P2PR8, PRPF8, SF1, U2AF2, CDX2, CDKN2C, CDKN2A, CDKN1B, BAX, BCL6, BRINP3, CD58, CDKN2B, CEBPG, CHD1, HNRNPK, ITPKB, MEF2B, PAG1, PTK2B, SF3A1, CDK12, CDH17, CDH11, CDH10, DNMT1, DUSP22, EBG1, EIF4A1, ELANE, EPOR, GLIS2, IKZF2, KLF2, MFHAS1, PLCG2, SEMA6A et SMC3.

8. Procédé selon la revendication 1, dans lequel le cancer est choisi dans le groupe constitué par : le cancer gastro-intestinal, le cancer de la prostate, le cancer des ovaires, le cancer du sein, le cancer de la tête et du cou, le cancer du poumon, le cancer du poumon non à petites cellules, le cancer du système nerveux, cancer du rein, cancer de la rétine, cancer de la peau, cancer du foie, cancer du pancréas, cancer génito-urinaire, cancer colorectal, cancer du rein, cancer hématologique, maladie hématologique, hémopathie maligne, maladie résiduelle minime et cancer de la vessie.

9. Procédé selon la revendication 1, dans lequel le profil génétique est généré par:
(a) déterminer une fréquence de la ou des mutations génétiques dans l'échantillon de tissu; et
(b) sélectionner la ou les mutations génétiques à inclure dans le profil génétique, la fréquence de la ou des mutations génétiques étant supérieure à un seuil;
ou dans lequel le profil génétique est généré par:
(a) déterminer un emplacement de la ou des mutations génétiques sur un chromosome; et
(b) sélectionner la ou les mutations génétiques à inclure dans le profil génétique, la ou les mutations génétiques étant situées sur différents chromosomes.
